# EUROPEAN PATENT SPECIFICATION

(11) **EP 1 507 562 B1**
(45) Date of publication and mention of the grant of the patent: **24.03.2010**
(21) Application number: 03730342.7
(22) Date of filing: 28.05.2003
(51) Int. Cl.: A61K 51/10, A61P 35/00

(54) **COMPOSITIONS FOR RADIOIMMUNOTHERAPY OF BRAIN**
ZUSAMMENSETZUNGEN FÜR DIE RADIOIMMUNTHERAPIE VON GEHIRN-TUMOREN
COMPOSITIONS DESTINEES A LA RADIO-IMMUNOTHERAPIE DES TUMEURS DU CERVEAU

(30) Priority: 29.05.2002 US 383617 P
(43) Date of publication of application: 23.02.2005
(73) Proprietor: Immunomedics, Inc., Morris Plains, NJ 07950 (US)
(72) Inventor: GRIFFITHS, Gary, L., Morristown, NJ 07690 (US); GOLDENBERG, David, M., Mendham, NJ 07945 (US)
(74) Representative: Bohmann, Armin K.
(86) International application number: PCT/GB2003/002334
(87) International publication number: WO 2003/101495

(56) References cited:
- WO-A-94/05203
- WO-A-99/66951
- WO-A-03/059397
- US-A- 4 824 659
- KARACAY H ET AL: "Experimental pretargeting studies of cancer with a humanized anti-CEA x murine anti-[In-DTPA] bispecific antibody construct and a (99m)Tc-/(188)Re-labeled peptide." BIOCONJUGATE CHEMISTRY. 2000 NOV-DEC, vol. 11, no. 6, November 2000 (2000-11), pages 842-854, XP002260850 ISSN: 1043-1802
- ROSSI EDMUND A ET AL: "Development of new multivalent-bispecific agents for pretargeting tumor localization and therapy." CLINICAL CANCER RESEARCH : AN OFFICIAL JOURNAL OF THE AMERICAN ASSOCIATION FOR CANCER RESEARCH. 1 SEP 2003, vol. 9, no. 10 Pt 2, 1 September 2003 (2003-09-01), pages 3886S-96S, XP002259779 ISSN: 1078-0432
- KARACAY H ET AL: "Pretargeting for cancer radioimmunotherapy with bispecific antibodies: role of the bispecific antibody's valency for the tumor target antigen." BIOCONJUGATE CHEMISTRY. 2002 SEP-OCT, vol. 13, no. 5, September 2002 (2002-09), pages 1054-1070, XP002259780 ISSN: 1043-1802
- KARACAY H ET AL: "Pretargeting with bispecific antibody as a method for improved imaging of pancreatic cancer." JOURNAL OF NUCLEAR MEDICINE, vol. 44, no. 5 Supplement, May 2003 (2003-05), page 176P, XP0008023949 & 50TH ANNUAL MEETING OF THE SOCIETY OF NUCLEAR MEDICINE; NEW ORLEANS, LA, USA; JUNE 21-25, 2003 ISSN: 0161-5505
- BIOLOGICAL ABSTRACTS, vol. 60, 1998, Philadelphia, PA, US, SANTOS A.D. AND PADLAN E.A.: "Development of more efficaceous antibodies for medical therapy and diagnosis" page 169
- COLOMA J. ET AL.: "Transport Across the Primate Blood-Brain Barrier of a Genetically engineered Chimeric Monoclonal Antibody to the Human insulin Receptor" PHARMACEUTICAL RESEARCH, vol. 17, no. 3, 2000, pages 266-274,
- NEUWELT A. ET AL.: "Delivery of Melanoma-associated Immunoglobulin Monoclonal antibody and Fab Fragments to Normal Brain Utilizing Osmotic Blood-Brain Barrier Disruption" CANCER RESEARCH, vol. 48, 1988, pages 4725-4729,

## Description

### BACKGROUND OF THE INVENTION

Cancers of the brain and central nervous system (CNS) are notoriously difficult to treat and are usually lethal. Astrocytomas are marked by a predilection for early and rapid infiltration into surrounding brain tissue that is primarily responsible for the failure in controlling these cancers. The most malignant stage of astrocytoma is glioblastoma multiforme (GBM) with survival times less than two years for most patients. A second and very important group of brain cancer patients are those with primary tumors that originate outside the CNS, but who have developed metastatic lesions to the brain. These include a large number of patients with primary cancers of the breast, lung, kidney, and prostate, as well as melanoma, and prognoses for such patients are extremely bleak. Indeed, the incidence of metastatic brain cancers is ten-fold higher than the incidence of primary brain cancer. At present, brain metastases are estimated to occur in 20-40 % of cancer patients with over 170,000 cases per year in the USA alone.

Surgical debulking and external beam radiation remain mainstays of treatment protocols whereas chemotherapy is rendered particularly difficult by the restricted systemic access to tumor sites by the blood brain barrier. External beam radiation can be stereotactically targeted to known tumor sites in the CNS and is often used against single or solitary brain lesions. However, such an approach does not address the issue of rapid infiltration of tumors and the regrowth of tumors following infiltration. In addition the method cannot be used to treat micrometastases. Surgical approaches face the same problems, and usually involve the removal of a greater margin of normal tissue.

Surgery followed by brachytherapy is a common way to attempt to overcome the problem of residual disease, and iodine-125 and iridium-192 are the most common nuclides used in radioactive seed implantation. It would be a useful advance if therapeutic agents could delivered in a manner that would decrease the adverse side effects associated with a treatment regime. One way of measuring the effectiveness of a treatment is with the use of a therapeutic index. The therapeutic index is defined as a ratio of a drug dose that produces an adverse side effect to the dose that causes the desired effect. There is a need for methods of treating brain cancer that have an increased therapeutic index compared to current methods.

Radioimmunotherapy (RAIT) with monoclonal antibodies (mAbs) is a particularly promising treatment for various types of cancers. Several different treatment methodologies have been developed based on radiolabeled antibodies targeted to antigens that are over-expressed in brain tumors, such as tenascin and epidermal growth factor receptor. Impressive results were obtained using such RAIT methodologies in comparison to standard treatment modalities (Riva et al, Eur. J. Nucl. Med., 27:601-609, 2000, and Akabani et al., Int. J. Radiat. Oncol. Biol. Phys., 46:947-958, 2000; etc).

However, standard RAIT methods suffer from the disadvantage that when a radiolabeled mAb is injected into a cancer patient a finite amount of time is needed for the radioimmunoconjugate to both maximize in tumor target tissue, and clear from background tissues and circulation. During this time, which is quite long for an intact radiolabeled immunoglobulin IgG and somewhat shorter for radiolabeled IgG fragments and sub-Fab' fragments, the patient is exposed to non-disease-targeted radiation. This non-targeted radiation, primarily received during the mAb localization phase, translates directly to radiotoxicity. This, in turn, limits the total amount of radiolabeled mAb that can be administered, preventing dose escalation to achieve optimal RAIT.

In an approach called indirect targeting or pretargeting, bispecific antibodies (bisAbs) have been used in the past with systemic radioimmunotherapy in a two-step approach. First, a non-radiolabeled bispecific antibody directed against a tumor antigen and against a radionuclide hapten conjugate is administered and allowed to localize and maximize at the tumor while clearing circulation and normal tissue. At a later time, a radiolabeled bivalent hapten is given that localizes to bisAb localized at tumors, but otherwise clears the system relatively completely and very rapidly via the urine. Whereas with a directly labeled antibody the living system is exposed to radionuclidic decay during a long period of 1-7 days while tumor uptake of the radiolabeled antibody maximizes, with a bisAb delivered radionuclide the system is only exposed to non-tumor-targeted nuclide for minutes to hours.

Furthermore, the number of antigen sites found on the tumor finitely limits the radiation dose that is localized to a tumor. In addition to this absolute limitation there is also a practical limitation in that not every antibody molecule is associated with a radionuclide molecule. This means that most of the tumor antigens are bound by a mAb molecule that is not carrying a radioactive payload. Without internalization and/or recycling, if one in ten mAb molecules carry a radionuclide atom, then only one in ten antigen sites can be targeted with a radionuclide. One-in-ten mAb molecules bearing a radionuclide is in fact a very good mAb-to-radionuclide ratio in practical terms, as often the ratio can be one-in-one hundred or even more. For instance, when one considers a sample of mAb labeled with the therapeutic radionuclide rhenium-188 at 1 mCi per mg of protein, about one in two hundred mAb molecules is actually associated with a radioactive Re-188 atom. Clearly, there would be an improvement in RAIT if more radionuclide could be directed to the antigen sites where it is needed, without unwanted blockade of the limited numbers of antigen sites on those tumors.
Coloma M. J. et Al., Pharm Res., 17, 2000, 266-274 discloses brain imaging with [¹¹¹In]-chimeric HIRNMAb on an anesthetized Rhesus monkey.
Neuwelt E. A. et Al., Can. Res. 48, 1988, 4725-4729 discloses delivery of melanoma-associated Immunoglobulin monoclonal antibody and Fab fragments to normal brain utilizing osmotic blood-brain barrier disruption.
Karacay H. et Al., Biocon. Chem. 2000, 11, 842-854 discloses experimental pretargeting studies of colorectal cancer with a two step-pretargeting method employing anti-CEA x murine anti-[-DTPA] bispecific antibody construct and a ^{99m}Tc/188Re-labeled peptide. WO9966951 published on 29.12.1999 deals with the use of bi-specific antibodies for pretargeting in diagnostic and therapeutic applications

Another major problem that still exists with RAIT protocols is that absolute tumor uptake of mAb as a percentage of the dose given is still usually very low in a clinical setting, often 0.01 to 0.00001 % injected dose per gram of tissue. Thus, a very small portion of the radioactive immunoconjugate that is injected is actually localized to the target tissue, while a majority distributes throughout normal tissues, leading to tissue damage. In addition, since tumor-associated antigens are upregulated, rather than tumor-specific compared to normal tissues, there are also a finite number of such upregulated sites available in tumors. The presence of large amounts of antigen in normal tissue will decrease the specificity of an antibody directed against a cancer that also expresses that same antigen. Furthermore, mAbs are delicate biological molecules that are readily impaired in their ability to bind to their antigenic targets if over-loaded or subjected to harsh conditions related to chemistry or radiolytic events or effects associated with systemic administration.

There is currently a need in the art for a methodology using an antibody to treat brain cancer, which has an improved therapeutic index relative to current methodologies. There is also a need for a methodology of administering a therapeutic antibody for the treatment of brain cancer, which minimizes antibody degradation and the loss of antibody activity.

### SUMMARY OF THE INVENTION

The present invention relates to the use of a multispecific antibody according to present claim 1. Tumors that are treatable by the present invention are brain tumors. A tumor is a neoplastic growth that can be benign or malignant. Cancer is a malignant tumor or neoplasm.

The present invention relates to optimizing treatment for brain tumors by maximizing the delivery of the therapeutic agent to the target. Optimizing therapeutic agent to the target site is particularly beneficial when the target antigen is sparsely expressed. In optimizing the delivery of the therapeutic agent to the target site, it is desirable to have the carrier of the therapeutic agent carry at least one therapeutic agent so that any antibody that binds to a target antigen will likely deliver one or more therapeutic agents. To achieve such targeting, it is often desirable to administer the targeting antibody, active agent, or both as close as possible to the target tumor. Preferred forms of administration include intracavity administration, such as intracranial and intratumoral administration, as well as intrathecal administration. Other forms of administration include intraaterial or intravenous administration, when the blood brain barrier is compromised. Any reference in this application to the term therapeutic agent is meant to refer to radionuclide.

The multispecific antibody can be administered prior to the radioconjugate or the two components can be premixed and administered as an immune complex.

### DETAILED DESCRIPTION OF THE INVENTION

In studying methods to improve the therapeutic index of antibody mediated therapy for the treatment of a tumor in the brain, the inventors developed a multispecific antibody comprising a targeting arm that binds a brain tumor antigen and a capture arm that binds a recognition moiety. A multispecific targeting agent is an agent that has at least one tissue targeting component and at least one secondary targeting component that recognizes a hapten. For simplicity, the multispecific antibody may be referred to illustratively as a bisMab herein but it should be understood that multiply specific as well as bispecific Mabs are intended, unless otherwise specifically noted.

The inventors also provide a methodology for improving the therapeutic index of an antibody-mediated treatment of brain tumor. This methodology involves treating a subject with brain cancer by administering the targeting agent regionally or locally, e.g. by routes such as intrathecally, intracranially, or intratumorally, whereby maximum brain tumor uptake is achieved. Other routes, such as systemic administration, may be utilized as well. For example, particularly in the case where the blood brain barrier is compromised, one would expect that intravenous and intraarterial routes would be useful. A multispecific antibody such as one comprising a targeting arm that binds a brain cancer antigen and a capture arm that binds a recognition moiety as in claim 1 can be administered prior to a therapeutic agent comprising the recognition moiety that is bound by the capture arm of the multispecific antibody. Alternatively, the antibody and therapeutic agent can be administered as an immune complex.

As stated above, there are a finite number of antigen binding sites available, and as is well-known, tumor uptakes of radiolabeled antibodies given systemically in a clinical setting are very low in terms of absolute amounts localized to tumors. Administration by intravesical, intrathecal, intraarterial, or intratumoral routes advantageously increases the absolute uptake, since relative tumor uptakes are higher when these routes of more localized administration are used. Another aspect of the present invention is combining these administrative routes with compositions of matter wherein each molecule of injected multispecific antibody carries approximately at least one radionuclide atom to its intended target. This can be accomplished in several ways as described herein.

The use of claim 1 may further comprise (a) radiolabeling a solution of the radionuclide carrier with the therapeutic radionuclide, wherein the carrier is present in excess of the therapeutic radionuclide and a therapeutic radionuclide carrier conjugate is thereby formed; (b) substantially removing from the solution carrier that has not complexed or bound to the therapeutic radionuclide; (c) providing the multispecific antibody as in claim 1 and (d) administering sequentially or together to the patient the multispecific antibody and the radionuclide carrier, wherein the radionuclide carrier that binds to the multispecific antibody and a targeting arm or targeting arms of the multispecific antibody form an antigen-antibody complex at the target cell. This can also include prior to the administering of (d): mixing the multispecific antibody and the therapeutic radionuclide conjugate so that substantially all multispecific antibodies are coupled with at least one therapeutic radionuclide conjugate. This can be done by mixing an excess of therapeutic radionuclide conjugate with a multispecific antibody of claim 1. After administration, the invention can further comprise allowing the multispecific antibody to bind to the tumor and to clear from circulation. The therapeutic radionuclide carrier is in a substantially equimolar ratio with respect to the multispecific antibody bound to the target tumor.

The use of claim 1 may further comprise (a) developing the multispecific antibody of claim 1 so that it is specific to a conjugate of the therapeutic radionuclide and the therapeutic radionuclide carrier, wherein the multispecific antibody has an affinity for the therapeutic radionuclide carrier conjugate that is approximately 100X or more higher than its affinity for the non-radiolabeled therapeutic radionuclide carrier alone; (b) radiolabeling a solution of the therapeutic radionuclide carrier with the therapeutic radionuclide, wherein the carrier is present in excess of the therapeutic radionuclide and a therapeutic radionuclide carrier conjugate is thereby formed; (c) adding the multispecific antibody to the solution; (d) optionally removing from the solution therapeutic radionuclide carrier that has not bound to the multispecific antibody; and (e) administering to the patient the coupled multispecific antibody and the therapeutic radionuclide carrier, wherein the therapeutic radionuclide carrier and the multispecific antibody are administered in approximately a 1:1 ratio or in any way to maximize the amount of radion nuclide delivered to the patient. The (d) removing can be done can be performed via an ion-exchange column or size-exclusion column, hydrophobic interaction column, or a chemically activated column that is capable of reacting with a non-complexed chemical moiety on the therapeutic radionuclide carrier.

The use of claim 1 may further comprise (a) radiolabeling a solution comprising a chelate with the therapeutic radionuclide, wherein the chelate and the radionuclide are optimized to deliver substantially at least one chelated radionuclide for every multispecific antibody of claim 1 and a therapeutic radionuclide chelate conjugate is thereby formed; (b) binding to the conjugate a non-radiolabeled multispecific antibody; and (c) administering to the patient the coupled multispecific antibody and the therapeutic radionuclide conjugate, wherein the therapeutic radionuclide conjugate and the multispecific antibody are administered to deliver substantially at least one chelated radionuclide for every multispecific antibody.

The use of claim 1 may further comprise (a) providing the therapeutic radionuclide carrier, wherein therapeutic radionuclide carrier has a thiol group; (b) binding the thiol group of the therapeutic radionuclide carrier to a thiol-binding radionuclide, whereby a radionuclide-carrier conjugate is formed; (c) passing the mixture of bound and unbound therapeutic radionuclide carrier over a substrate activated with a thiol-reactive chemical moiety, whereby carrier with thiol groups remaining unbound to radionuclide reacts with the substrate and is retained while therapeutic radionuclide carrier with radionuclide-bound thiol is eluted; (d) administering to a patient in need thereof, a pharmaceutically effective amount of the multispecific antibody and the therapeutic radionuclide carrier conjugate, wherein the therapeutic radionuclide carrier conjugate binds to the multispecific antibody of claim 1. The thiol-reactive chemical moiety can comprise a haloacetate or a maleimide group.

The invention can further comprise the multispecific antibody of claim 1 the therapeutic radionuclide carrier, and the radionuclide, administered in approximately a 1:1:1 ratio or better (that is where the approximately at least or more therapeutic radionuclide per multispecific antibody), wherein the composition is substantially free of the therapeutic radionuclide carrier that is not bound to the radionuclide.

### Routes of Administration

Preferred routes of administration include intracavity routes, such as intrathecal, intracranial, or intratumoral. It is again noted that the comprised blood brain barrier may indicated intraaterial or intravenous administration.

The bisAb and the RAIT agent can be premixed prior to injection, but this is not necessary, and the two agents can be given separately. Indeed, there are instances where it is desirable to give the agents separately. In one embodiment, the bisAb is given by an intraarterial route, for example via the caroid artery, and allowed to passage through to its targets in the brain and CNS while thoroughly clearing systemically. During this phase no radionuclide is associated with the bisAb and toxicity to neuronal tissue is thereby reduced when compared to a premixed methodology. At an appropriate time later the radiolabeled secondary recognition hapten is given, for instance intrathecally, such that it readily diffuses into surrounding brain tissue and is recognized and bound by the pretargeted bisAb. In a particularly preferred embodiment, the bisAb can be radiolabeled with a tracer nuclide, such as iodine-131 or technetium-99m, using known methods, to accurately estimate the amount of bisAb present at tumor sites, and remaining in circulation, and then estimate the amount of secondary recognition unit/therapy nuclide that needs to be given and the optimal time for its administration.

### Valency of Haptens

In the below examples, radiolabeled haptens are described as monovalent, for simplicity and understanding. Monovalency is quite acceptable, particularly when using secondary antibodies to radiolabeled haptens that have a high affinity, typically 10⁻⁹ and above. Routine experimentation can determine the level of affinity needed for the interaction of the secondary MAb recognition arm and the radiolabeled hapten. Typically, those binding pairs will have fast on-rates (Kₒₙ) and slow off rates (K_{off}), or fast rates of formation and slow rates of dissociation.

However, the recognition haptens may have a higher valency than one toward the recognition arm of the bisAb. Bivalent recognition haptens are particularly well known in the art, and in systemic administrations it is well known that bivalency confers an advantage toward tumor retention of radiolabeled secondary hapten localized via a pretargeted bisAb. In addition, bivalent hapten recognition units such as those with more than one chelate can be useful in increasing the achievable specific activity when conversion to the radiometal-labeled bivalent hapten is done. This is a useful preferred way to operate under the third embodiment related to monovalent species, discussed above. For instance, agents such as DOTA-X-DOTA, where X = any kind of standard linking agent are useful in this invention. Suitable -X- include a peptide, an alkyl or aryl group or a carbohydrate backbone.

### Polymer Haptens

The same principles can be extended to recognition haptens of even greater valency, such that polymer backbones are used to carry, for instance, multiple chelate moieties, each capable of binding one atom of radiometal. More useful structures similar to these types of agents are exemplified in US Provisional Patent Application 60/308,605, filed on July 31, 2001,. Even small polymers bearing only 2-10 chelates need to be added in very small amounts in order to bind all available metal ions in a solution of carrier-free radionuclide, once the metal chelate and the radionuclide to be bound are chosen judiciously.

With any of the secondary recognition units, the recognition does not necessarily involve the chelating agent that is carrying the radiometal. It is merely preferred in several embodiments. In the embodiments related to the use of polymeric carriers of metal chelates it is not preferred. Indeed the opposite is preferred, because the recognition unit to be bound by the bisAb is better a separate entity from the radionuclide-bearing moiety; such as a metal-chelate complex. In the latter case, where the greater concentration of the added chelate per mole of polymer is being relied on to ensure that ultimately each bisAb will be able to bind at least one radionuclide atom, a preferred polymeric carrier species has multiple metal-chelating groups but only one or two recognition units. This is to ensure that too many recognition units are not present that can cause premature clearance and metabolism of multi-cross-linked bisAb-polymer complexes.

### Antibody Fragments and Multispecific Antibodies

Multispecific antibodies can be Fab' x Fab' species or IgG species wherein one arm binds to tumor target and one arm to secondary recognition unit. They can be prepared by chemical cross-linking or via somatic mutation using quadromas. Multi-specific antibodies with valences of greater than one for either the tumor antigen and/or the recognition hapten are within the scope of the invention. For instance, species such as IgG-1 x IgG-2 or IgG x (scFv)₂ can be prepared wherein two antigen targeting and two secondary recognition sites are present. These types of agents can be prepared using now standard techniques of cloning. Smaller tumor antigen and secondary recognition multispecifics can be made and used, down to the minimal molecular recognition units, without deviating from the essential spirit of the current invention. Similarly, agents that are bivalent x monovalent such as F(ab')₂ x Fab', wherein the F(ab')₂ targets tumor antigen and the Fab' targets the secondary recognition unit, or vice versa, are within the scope of the invention. In the case of multivalent species such as F(ab')₂ x Fab', multi-specificity is also possible. Thus, with the F(ab')₂ x Fab' example, when the tumor-targeting arm is represented by the F(ab')₂ unit, each of those individual arms can be directed against separate tumor antigens, such as carcinoembryonic antigen (CEA) and epidermal growth factor receptor (EGFR), or tenascin and EGFR. Also, different arms of the multispecific can be targeted against different epitopes of the same antigen. The term multi-specific as used herein is meant to include bispecific.

MsAbs can include antibody fragments, subfragments and combinations thereof. The antibody fragments are antigen binding portions of an antibody, such as F(ab')₂, F(ab)₂, Fab', Fab. The antibody fragments bind to the same antigen that is recognized by the intact antibody. For example, an anti-CD22 monoclonal antibody fragment binds to an epitope of CD22. The msAbs of the present invention also include IgG x IgG, IgG x F(ab')₂, IgG x Fab', IgG x scFv, IgG x sFv, F(ab')₂ x F(ab')₂, Fab' x F(ab')₂, Fab' x Fab', Fab' x scFv, Fab' x sFv, (sFv x sFv)₂. sFv x sFv, and scFv x scFv bi-specific monoclonal antibodies (bismAbs). Also, species such as scFv x IgG x scFv and Fab' x IgG x Fab', scFv x F(ab')₂ x scFv and Fab' x F(ab')₂ x Fab' are included. Most preferably, site-specific attachment sites on the IgG or F(ab')₂ of one or both monoclonal antibodies (mAbs) can be utilized, such as an engineered carbohydrate or an engineered or liberated free thiol group. Since these mAbs are dimeric they can be coupled with two moles of the second mAb. For instance, a mAb directed towards carcinoembryonic antigen (CEA), anti-CEA F(ab')₂, having an engineered light-chain carbohydrate can be oxidized and converted using a hydrazide-maleimide cross-linker to a derivatized anti-CEA F(ab')₂ having at least one pendant maleimide group per each light chain. This species is coupled to an anti-chelate Fab'-SH at a 1:2 molar ratio, at least, such that an anti-chelate-Fab' x anti-CEA-F(ab')₂-anti-chelate Fab' conjugate is produced. The resultant msAb is bivalent with respect to the target tissue and the polymer conjugate. At their smallest, msAbs constructed with peptide molecular recognition units directed against each specificity, including also diabodies, triabodies, tetrabodies, quintabodies. It is further understood that the use of the term "msAb" in the present disclosure encompasses multi-specific antibodies and multi-specific antibody fragments.

The term "antibody fragment" also includes any synthetic or genetically engineered protein that acts like an antibody by binding to a specific antigen to form a complex. For example, antibody fragments include isolated fragments, "Fv" fragments, consisting of the variable regions of the heavy and light chains, recombinant single chain polypeptide molecules in which light and heavy chain variable regions are connected by a peptide linker ("sFv proteins"), and minimal recognition units consisting of the amino acid residues or related peptides that mimic the hypervariable region.

The msAbs of the current invention may be monoclonal or polyclonal in nature, but preferably monoclonal. Furthermore, the targeting arm and the capture arm of the msAb may be monoclonal or polyclonal in nature. Preferably, either the target arm or the capture arm is monoclonal. Most preferably, the target arm and the capture arm are both monoclonal.

The msAb of the current invention may be engineered to possess a label. Examples of labels that the msAb may possess include a labeling ligand such as the biotin-streptavidin complex and radioisotopes. Advantageously, the msAb of the current invention is radiolabeled to facilitate tracking of localization and clearance.

MsAbs useful in the current invention are also understood to encompass msAbs with more than one targeting arm such as a F(ab')₂ x Fab' fragment. Thus, one arm can be targeted against the recognition hapten with two arms directed toward a tumor-associated antigen, or *vice versa*. In addition, the F(ab')₂ part of the F(ab')₂ x Fab' fragment (assuming the Fab' part is directed against the radiolabeled hapten) can be directed against two distinct epitopes on the same antigen (e.g., CEA) or two distinct antigens (e.g., CEA and MUC1). It, itself can thus be multispecific in terms of targeting ability, with one Fab' or sFv arm directed against one tumor antigen and one directed against a second tumor antigen on target tissue. In addition, one targeting arm of this F(ab')₂ or (sFv x sFv)₂ sub-species can be directed against a tumor antigen while the second targeting arm is directed against a separate type of antigen, such as a vascular antigen epitope, present on a brain tumors.

Diabodies and triabodies are also useful for the present invention and are further discussed in U.S. Provisional Application Nos. 60/314,881 filed December 21, 2001, 60/328,835 filed October 15, 2001, 60/342,103 filed December 26, 2001, 60/345,641 filed January 8, 2002.

Also useful for this invention are the bispecific fusion proteins described in U.S. Application Nos. 09/911,610, filed July 25, 2001, 09/337,756, filed June 22, 1995 and 09/823,746, filed April, 3, 2001. Other antibodies and useful compositions and method for the present invention include I253A, and the subject matter disclosed in U.S. Provisional Application 60/361,037, filed March 1, 2002.

### Antibodies and Anitgenic Targets

Various antibodies known in the art can be used in the targeting of brain cancers. The present invention is preferably for primary and secondary (metastatic) brain tumors, and depending on the tumor type, different antibodies and antibody combinations would be selected. Antigenic targets useful within the scope of the invention include tenascin, epidermal growth factor receptor (EGFR), platelet derived growth factor receptor (PDGFR), fibroblast growth factor receptors (FGFRs), vascular endothelial growth factor receptors (VEGFR), and various gangliosides.

The present invention is also useful for treating metastases of other tumors, such as carcinomas and melanomas, to the brain. Antibodies to antigens associated with tumors that metastasize to the brain are carcinoma-associated antibodies, such as antibodies to carcinoembryonic antigen (CEA), MUC1, TAG-72, epithelial glycoprotein (EGP), HER, PSMA, MAGE, B72.3, CD44, and S100. As useful in the present invention is antibodies against, EGP-1 (RS-7), CD-74 (LL1 for melanoma) and CEA, as diclosed in U.S. Patents 4,348,376, 4,818,706, 4,900,684, 5,874,540, U.S. Applications 09/253,744 and U.S. Provisional Applications 60/360,229 filed March 1, 2002, and 60/360,259 filed March 1, 2002. The most prevalent tumors that metastasize to the brain are lung cancer, prostate cancer, breast cancer, and melanoma. In addition, because virtually any cancer can spread to the brain or CNS, including lymphomas and leukemias, even more antigen targets, such as in lymphomas and leukemias, cf. CD20, CD22, and other B-cell antigens, can be useful for the present invention. Antibodies against CD20 are cited in U.S. Provisional Application 60/356,132 filed on February 14, 2002, antibodies useful against CD22 are disclosed in U.S. Patents 6,183,744, 6,306,393, As in the case of primary brain cancers, a preferred method for treating these metastatic tumors is by using suitable combinations of antibodies against more than one antigen type. In the case of metastases, however, it may be preferred to also treat systemically, e.g., intravenously, since it is usual that when there are metastases to the brain, there are also metastases to other organs in the body. For a review of antibody targeting, see Goldenberg DM, Targeted therapy of cancer with radiolabeled antibodies, J Nucl Med 2002; 43 (5):693-713. Additional antibodies include products of oncogenes, and antibodies against tumor necrosis substances, such as described in patents by Epstein et al. (U.S. Pat. Nos. 6,071,491, 6,017,514, 5,019,368 and 5,882,626).

### Antibody Preparation

Antibodies to secondary recognition haptens can be prepared using standard methods of immunologic priming followed by generation of hybridoma clones producing monoclonal antibodies of interest. In this manner, various specific antibodies have been made and produced in bulk, and these include antibodies to the metal-chelate complexes indium-diethylenetriaminepentaacetic acid (In-DTPA), and yttrium-1,4,7,10-tetraazacyclododecane-N,N',N",N"'-tetraacetic acid, (Y-DOTA), and to other diverse species such as histamine-succinyl-glycine (HSG), biotin and fluorescein. The current invention includes in its scope any antibody to any secondary recognition hapten, including multispecific antibodies that can bind to any epitope on a large structure, such as a polymer. Specificities and affinities of the tumor targeting and the secondary recognition mAb can be pre-selected using standard methods of phage display, and human msAbs of desired properties obtained thereby. Specific antibodies can be affinity matured by techniques known in the art in order to enhance affinity and on- and off-rates.

MsAbs of the current invention are prepared by well-known methods using chemical linkages, somatic methods, or by molecular biology derived expression systems, producing proteins in appropriate host organisms. It is to be appreciated that the source or the mode of production of the msAb is not central to the current invention. Thus the term msAb is herein intended to encompass any multivalent, multispecific, targeting antibody or fragment/subfragment, and specifically includes divalent x divalent and trivalent x monovalent and trivalent x divalent species, multispecific mini-antibodies, diabodies, triabodies, tetrabodies, quintabodies, and scFv x scFv tandems.

In a preferred embodiment the targeting msAb can be radiolabeled for easier quantitation of the amount taken up in the tumor tissue. This can be done by simple subtraction or it may be done using a well-known imaging technique, in either case, after elimination of the unbound radiolabeled msAb. Using a penetrating radionuclide, computed tomographic (CT) or single photon emission computed tomographic (SPECT), or positron emission tomographic (PET) imaging can be performed prior to administration of the radionuclide recognition hapten conjugate. In any event the purpose of this quantitation is to better gauge the amount of radiolabeled recognition hapten that is appropriate for a particular patient.

Recognition haptens of the current invention only need to have at least one epitope that is recognized by at least one arm of the pretargeted msAb. This is quite different from standard msAb RAIT protocols, wherein bivalent hapten binding is very important. When msAb RAIT therapy is performed systemically it has been shown that the recognition needs to be bivalent in nature. If it is monovalent, it does not bind well enough to pretargeted msAb to be retained for a long time in the tumor target. If it is tri- or higher valent then the risk is that formation of high molecular weight complexes in the serum will lead to premature clearance of the radiolabeled recognition hapten, primarily into the liver and spleen of the patient, resulting in poor tumor uptake and non-specific radiotoxicity. The current invention therefore encompasses recognition haptens of any valency to msAb from one upward, with no concern for the dual problems of poor retention and premature clearance.

Because of the issues discussed in the last paragraph, considerably more freedom can be applied to the design of recognition haptens for use in msAb-pretargeted RAIT. In the simplest form, a conjugate of the recognition hapten and the radionuclide can now be used since monovalent binding is useful within the scope of the invention. Examples of this are msAbs bearing an arm reactive with a metal complex of chelates such as DTPA or DOTA, anti-biotin mAbs for use with biotin-chelate conjugates, and anti-HSG mAbs for use with HSG-chelate conjugates. In these examples the metal is radioactive and bound strongly by the chelating agent. It is known that metal complexes of low molecular weight chelates can be prepared at near 1:1 ratios of metal to chelate, if the metal is purified appropriately and the chelate is chosen appropriately. Radiometals useful in the current invention include those that decay with particulate emission such as alpha and beta emitters, and/or with low energy gamma ray emission (Auger emitters). They include the following, in a non-exhaustive list: Sc-47, Ga-67, Y-90, Ag-111, In-111, Sm-153, Tb-166, Lu-177, Bi-213 and Ac-225. For radiolabeling, it should also be borne in mind that any of these metals can be initially complexed by an excess of a chelating agent, with the excess chelating agent then removed from the metal-chelate complex. The separation is usually based on an ion-exchange procedure since multiple negative charges on a chelate are neutralized after binding to a metal cation. Methods to perform such purifications have been described in the scientific literature.

Alternate radiometals that bind to thiol or thiol-amino containing ligands can also be used within the scope of the invention. These radiometals include, Cu-64, Cu-67, Pd-109, Ag-111, Re-186, Re-188, Pt-197, Bi-212, Bi-213 and Pb-212.

### Haptens

Haptens carrying non-metallic therapeutic radionuclides can also be used. For instance the recognition units epsilon-HSG-lysyl-tyrosine and HSG-tyrosine can be radioiodinated with the 1-125 or I-131 radionuclides, and the radioiodinated recognition units can be used after msAb pretargeting. Similar agents can be prepared using radioastatine, if a therapeutic alpha-particle emitting radionuclide is desired. Newer radioiodination agents have been designed that produce a non-metabolizable form of radioiodine that is retained in cells after intracellular processing. A variety of such agents have been described in the scientific literature and they can be used to prepare conjugates of recognition haptens with residualizing radiohalogen sub-units. The preparation of conjugates of the recognition hapten and the moiety that actually carries the radionuclide uses standard techniques and methods of organic chemistry. Any appropriate chemical linkage can be used, exemplified carboxyl to amino to produce an amide bond, thiol to halocarbon to produce a thioethers bond, amino to aldehyde to produce an imine bond, optionally reducible to a secondary amino bond, etc. When appropriate short linkers can be used, such as a diamine used to link a carboxyl-containing nuclide carrier (e.g. metal-DTPA) and a carboxyl-containing recognition unit (e.g. histamine-succinyl-glycine). It is understood that these general principles are applicable to all the conjugates that may be prepared for use in this invention.

Bivalent recognition haptens used in systemic msAb therapies are also useful with this intravesicular approach. Basically, any suitable chemical linkage can attach the two recognition haptens to each other. For instance, two recognition haptens linked by a short linear or cyclic peptide, as exemplified by:
Ac-Phe-Lys(DOTA)-Tyr-Lys(DOTA)-NH₂
DOTA-Phe-Lys(HSG)-Tyr-Lys(HSG)-NH₂
Ac-Phe-Lys(DTPA)-Tyr-Lys(DTPA)-NH₂DOTA-Phe-Lys(HSG)-D-Tyr-Lys(HSG)-NH₂
Ac-Lys(HSG)-D-Tyr-Lys(HSG)-Lys(Tscg-Cys)-NH₂
Ac-Cys-Lys(DOTA)-D-Tyr-Ala-Lys(DOTA)-Cys-NH₂

In these examples the DOTA or DTPA units can be radiolabeled with any of the same therapeutically useful radiometal radionuclides listed above that prefer oxygen-nitrogen ligands. Likewise, the chelate Tscg-Cys- (thiosemicarbazonylglyoxyl-cysteine-) is designed to be labeled with therapeutic radiometals that prefer thiol-nitrogen ligands. The peptides can be designed with tyrosyl residues already incorporated so that they can be readily iodinated with I-125 or I-131. Peptides that contain more than one carrier site that can accept a radionuclide can be double labeled, for instance with radioiodine and with a radiometal. Peptides can be chosen to be resistant to enzymes, such that they contain D-amino acids, and are N-terminal acylated and C-terminal amidated. The above species can be used with msAbs having anti-DTPA, anti-DOTA or anti-HSG secondary recognition arms, as appropriate. The same recognition units can also be readily attached to templates that are non-peptide in nature. For instance simple diamines can be doubly substituted with DTPA or DOTA moieties. An appropriately substituted diamino-sugar template can be doubly substituted with DOTA or DTPA in a similar manner.

More than two recognition units can also be use in the practice of the invention. Most preferably this is done when the recognition unit is also an integral part of the radiotherapy agent, for example, a yttrium-90-DOTA chelate complex. Such complexes can be multiply substituted onto polymeric carriers. The polymeric carriers that carry agents such as yttrium-90-DOTA and are used in this invention are preferably administered intravesically, since there is then much less concern about non-specific tissue uptake, and metabolic clearance of large amounts of radionuclide into tissues such as the liver and kidney. In a preferred embodiment, the recognition unit and the radionuclide carrier are separated such that a polymer of the type [HSG]ₘ-polymer backbone-[DOTA-yttrium-90]ₙ is generated, where HSG comprises the recognition hapten. Preferably m = 1, while n = 10-100. In any event, the level of substitution of the recognition hapten is then held at 1-2 per polymer unit, while the level of the DOTA substitution is maximized per unit of polymer. This type of complex, freed from systemic pharmacokinetic concerns, can be readily super-loaded with Y-90. Since binding and recognition to tumor is via an HSG-containing msAb it can be ensured that every msAb pretargeted to the tumor will deliver at least one atom of yttrium-90 for therapeutic decay.

### Removal of Excess Antibody Prior to Administration

Prior to administration, the excess free antibody can be removed from solution. This can be performed via an ion-exchange column or size-exclusion column, hydrophobic interaction column, or a chemically activated column that is capable of reacting with a non-complexed chemical moiety on the therapeutic radionuclide carrier.

### Therapeutic/Diagnostic Radionuclide Agents

In the methods of the invention, the targetable construct may comprise one or more radioactive isotopes useful for treating diseased tissue. Particularly useful therapeutic radionuclides include ³²P, ³³P, ⁴⁷Sc, ⁶⁴Cu, ⁶⁷Cu, ⁶⁷Ga, ⁹⁰Y, ¹¹¹Ag, ¹¹¹In, ¹³¹I, ¹⁴²Pr, ¹⁵³Sm, ¹⁶¹Tb, ¹⁶⁶Dy, ¹⁶⁶Ho, ¹⁷⁷Lu, ¹⁸⁶Re, ¹⁸⁸Re, ¹⁸⁹Re, ²¹²Pb, ²¹²Bi, ²¹³Bi, ²¹¹At, ²²³Ra, ⁹⁰Y, and ²²⁵Ac. The therapeutic radionuclide preferably has a decay energy in the range of 20 to 6,000 keV, preferably in the ranges 60 to 200 keV for an Auger emitter, 100-2,500 keV for a beta emitter, and 4,000-6,000 keV for an alpha emitter.

### Preparation and Administration of Multispecific Radiolabeled Antibodies and Carrier Free Radioisotope

The therapeutic radionuclide carrier can comprise one or more of the following: diethylenetriaminepentaacetic acid, 1,4,7,10-tetraazacyclododecane-N,N',N",N"'-tetraacetic acid, and/or thiosemicarbazonyl-cysteinylglycine chelating agents. The therapeutic radiolabeled carrier can comprise a metal chelating agent.

Meares et al. (US Patent 5,958,374) describe a method of direct antibody radiolabeling, by first making a metal chelate complex with yttrium-90, or other M3 + cations, and purifying the M3+-chelate complex from unbound chelate by an ion exchange procedure. In the Meares method, the chelate M 3 + complex also bears an activated reactive chemical group to enable coupling of the M 3 + to the antibody targeting agent. Compositions similar to the intermediate reagents described by Meares can be prepared within the context of the present invention as follows. A derivatized DOTA made from reaction of a carboxyl group monoactivated DOTA and a primary amine, such as N-butylamine is used. This derivatized DOTA, termed DOTA-R is the species that will be labeled with yttrium-90, with the yttrium-90-DOTA-R further processed as described below.

First a multispecific antibody is prepared comprising at least one arm reactive against a brain cancer antigenic epitope, and at least one-second arm reactive against an anti-chelate secondary recognition arm. Such an agent is exemplified by an anti-EGFR x anti-yttrium-DOTA antibody. Yttrium-DOTA is yttrium-1,4,7,10-tetraazacyclododecane-N,N',N",N"'-tetraacetic acid.

Second a sample of carrier-free Yttrium-90-DOTA-R conjugate is prepared where R is a substituted moiety present to neutralize one of the four carboxyl groups of the tetra-acidic DOTA ring. Radiolabeling is accomplished by adding an excess of DOTA-R to a vial of yttrium-90 radionuclide. After a labeling period of from 5 minutes to 2 hours, the reaction mixture is applied to an ion exchange column, typified by a DEAE-cellulose anion exchange resin in acetate form. The electrically neutral complex yttrium-90-DOTA-R can be directly eluted from the anion exchange resin with water, while uncomplexed DOTA-R, being charged at 3- at near-neutral pH is retained on the resin. Thus is obtained a solution of carrier-free yttrium-90-DOTA-R.

The nature of R in the above embodiment is not critical and is chosen to remove the remaining negative charge on the DOTA ring that would remain after metal complexation. In a preferred embodiment R corresponds to an N-alkyl derivative such as N-propyl-, N-butyl-, N-pentyl-, etc. It is even possible to have R = H and leave one charge remaining on the DOTA ring, although this makes the ion exchange separation more delicate since a separation of a 0 charged from a 3- species is much easier than separation of a 1- charged species from a 3- species. Typically, R contains an amino group that reacts with one of the carboxyl groups of the DOTA ring to form a charge-neutral amide bond.

### Anti-Hapten Antibody Specific for Metal Chelate

The therapeutic radiolabeled carrier for the present invention can be a metal chelating agent. In this case, an anti-hapten antibody with specificity for a particular metal-chelate complex can be specifically developed. For instance, an antibody that has an affinity 2-4 orders-of-magnitude higher for a particular metal-chelate complex than the same chelate alone is particularly useful within the scope of the present invention. Using standard methods of antibody panning and affinity maturation, for instance, an antibody that reacts with much greater affinity with yttrium-DTPA (diethylenetriaminepentaacetic acid) compared to DTPA alone, is developed. For labeling, enough excess DTPA is added to the yttrium-90 therapeutic radionuclide to bind essentially all the nuclide, with the knowledge that subsequently added bisAb comprising the anti-yttrium-DTPA antibody will preferentially bind to the yttrium-90-DTPA chelate over the uncomplexed DTPA. A similar strategy can be used with any metal-chelate complex, including those based on rhenium-188, bismuth-212, bismuth-213, samarium-143, indium-111, and other carrier-free nuclides.

### Anti-Hapten Antibody Specific for Radioconjugate

It should be appreciated that the above secondary MAb production and use strategy is not restricted to a metal-chelate complex but may be also be used with covalent species. For instance iodine has several nuclides of possible utility for RAIT. Iodine is one of the largest elements, with an atomic number of 53, a nearly full outer shell of electrons, and an ionic radius > 2 Angstroms. The exquisite apseicificity of the antibody response can be used to distinguish between epitopes containing tyrosine and those containing iodotyrosine. Thus, the secondary MAb can be raised against iodotyrosine such that a bisAb secondary recognition arm raised against iodotyrosine will react exclusively with radioiodotyrosine derivatives and not with similar but unsubstituted tyrosine derivatives. Thus it is not necessary to remove unlabeled carrier after conjugation with the radionuclide. A similar strategy could be used to raise antibodies against astatine-containing immunogens, to be followed by RAIT with bisAb and a recognition unit containing that alpha-emitting radionuclide. Congugates and bifunctional ligands useful for the present invention include those disclosed in U.S. Patent No. 5,612,016.

### Efficient Chelating Agents

Certain chelating agents, such as DTPA, DOTA, and others are known to be such efficient metal scavengers that they can bind certain metals in highly dilute solutions, such that a molar equivalent of the radionuclide metal can be effectively bound by a molar equivalent of the added DTPA chelator. These metals include yttrium-90, samarium-153 and lutetium-177.

Also useful in the present invention are the binding ligands disclosed in U.S. Patent No. 6,126,916 and the chelating agents disclosed in U.S. Application 09/823,746, filed on April 4, 2001.

### Exploiting Differences in Chemical Reactivity

Another method for separating radioconjugates from unlabeled carriers relies on differences in the chemical reactivity of a chelating ligand after reaction with a radiometal. For instance thiol-containing ligands are used to bind radiometals such as rhenium-188, copper-67, copper-64 and silver-111, all useful radionuclides for radioimmunotherapy. Thiols react very readily with certain functional groups such as halogenoacetates and maleimides by substitution and addition reactions, respectively. These reaction properties can be used to prepare essentially carrier-free reagents by first binding a thiol-containing ligand to one of these nuclides and then passing the mixture of radiometal-bound and radiometal-free thiol-containing ligand over resins or beads activated with iodo-, bromo- or chloroacetates or maleimides, whereupon the radiometal-free thiol-containing ligands react, and the radiometal-bound thiol-containing ligand is eluted. Such an agent is represented by the general structure mercaptoacetylglycylglycylglycyl-Q , (MAG₃-Q) where Q is any group that is recognized by a secondary antibody recognition arm, if the secondary recognition arm is not raised against the MAG₃ or metal-MAG₃ moieties.

### Use with Other Therapies

The present invention has the advantage that it can be used with or without concomitant chemotherapy or external radiation. In certain cases, such as the case of a rapidly growing tumor, where there is an extracranial disease, the antibody of present claim 1 could be used with another therapy, such as chemotherapy, which can be given systemically.

### Example 1: Preparation of a Bispecific Antibody

(a) An anti-tenascin and an anti-hapten antibody termed 679 (murine; anti-histaminyl-glycyl-succinimidyl- (HSG-) moiety) are separately digested to F(ab')₂ fragments by incubation for one hour with 200 ug/mL of pepsin at pH 3.7, in acetate buffer. In each case the F(ab')₂ fragment is purified from reagents and side-products by size-exclusion and ion-exchange chromatography to yield products that are substantially pure 100,000 kD fragments.

(b) The F(ab')₂ fragments from the above pepsin digestions are separately incubated for one hour at 37°C in 0.1 M phosphate buffered 0.9% sodium chloride (PBS) buffer, pH 7.5, with 10 mM freshly-prepared L-cysteine. The reduced Fab'-SH fragments are separately purified by centrifugation on spin-columns containing G-50-80 Sephadex^{®}, equilibrated in sodium acetate buffer, pH 5.5. The product Fab' fragment antibodies are kept at 4°C prior to the cross-linking reaction.

(c) The 679-Fab'-SH fragment from b) above is reacted with a twenty-fold excess of the thiol-cross-linking agent *ortho*-phenyldimaleimide (OPD), dissolved in dimethyl sulfoxide, such that the final concentration of dimethyl sulfoxide in the activation reaction is 15%, and allowed to react for 30 minutes at 4°C. The product, 679-Fab'-S-linker-maleimide, is purified by centrifugation on a spin-column containing G-50-80 Sephadex^{®}, equilibrated in sodium acetate buffer, pH 5.5. The 679-F(ab')₂-S-linker-maleimide is mixed with a molar equivalent of the anti-tenascin-Fab'-SH and allowed to react at 4°C for 30 minutes. The desired product anti-tenascin-Fab'-linker-Fab'-679 (a Fab'₁ xFab'₂ bispecific antibody) is obtained pure by preparative size-exclusion high-performance liquid chromatography on a TSK-3000 (Tosohaas, Montgomeryville, PA), to remove low molecular weight contaminants and unreacted Fab' species.

### Example 2: Preparation of a Yttrium-90 Radiolabeled Bivalent Hapten

The mono-DOTA, di-HSG bivalent hapten peptide termed IMP 241 (DOTA-Phe-Lys(HSG)-D-Tyr-Lys(HSG)-NH₂. shown below, is radiolabeled with Y-90 using ⁻6 nmol of peptide and ⁻1 mCi of dried Y-90 chloride. Six microliters of 0.25 M ammonium acetate, pH 5.4, followed by 2.7 uL (5.94 nmol) is added to a 2.2 mM solution of IMP-241 in 0.25 M ammonium acetate, pH 5.4. The solution is heated for 30-40 min at 55°C using an aluminum block heater, then quenched with 10 mM DTPA (final conc.), heated for a further 10 minutes at the same temperature, and cooled. The solution is diluted with 40 uL of water, and mixed with 4.5 uL of 0.1 M aqueous triethylamine to raise the final pH to ⁻7.5. A similar labeling is performed with In-111 acetate instead of yttrium-90 acetate.

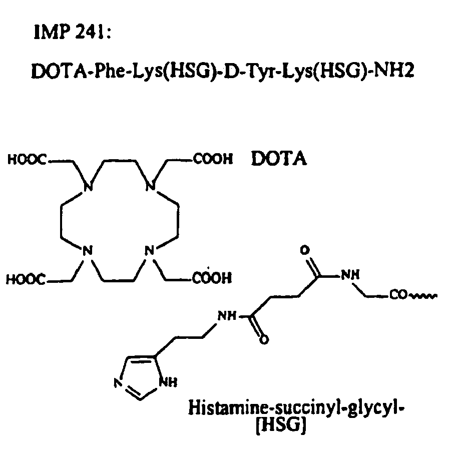

### Example 3: Preparation of a Carrier-free Yttrium-90 Radiolabeled Bivalent Hapten

The Y-90-IMP 241 from Example 2, above, is purified from non-U-90-containing IMP 241 on Dowex ag 1-x2 anion exchange resin using gravity flow, as follows. The radiolabeled solution is placed on 0.5 ml of the resin bed in a 1-ml syringe fitted with a 2-way stopcock (the flow stopped). After 1 minute, the solution is percolated through the resin bed to just near the top of the resin bed. The flow is stopped for another minute to allow resin contact, and then continued with 10 x 0.125 ml fractions of water. Most of the applied radioactivity is recovered in fractions 4-11. Using this approach a 100-fold depletion in the level of non-Y-90-containing peptide is achieved in the final product, resulting in a specific activity of 27,888 ci Y-90 per mmol of peptide. Since the specific activity of Y-90 itself is ⁻ 500 ci/mg (45,000 ci/ mmol), this corresponds to 0.6 mmol of Y-90 associated with each 1 mmol of peptide, or under two molecules of peptide per molecule of Y-90 radionuclide. A second passage through ag 1-x2 resin reduces the peptide-to-yttrium-90 ratios to very close to 1:1, if desired. The Y-90-IMP 241 is then ready for injection, or is diluted further for injection or infusion.

### Example 4: Preparation of a Rhenium-188 Radiolabeled Bivalent Hapten

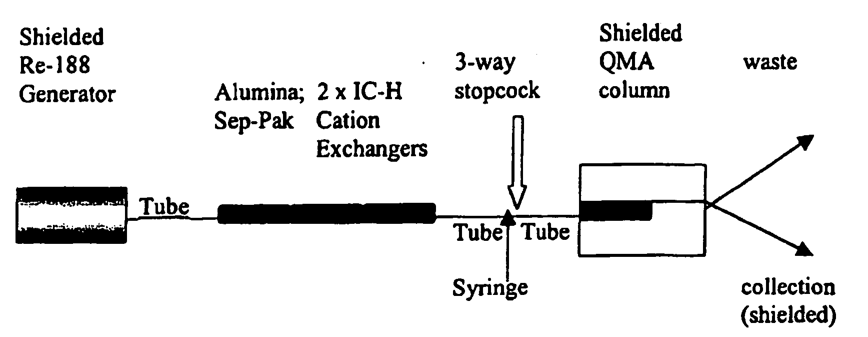

(a) A tungsten-188/rhenium-188 generator is set up for elution, purification and concentration of the rhenium-188 eluate, schematically, as shown above. The eluent is passed through a tube trough to four columns, set up in tandem: One alumina Sep-Pak cartridge (to adsorb breakthrough tungsten), two Alltech IC-H cation exchange columns (to adsorb acetate ions), and one Waters QMA light anion exchange column (to adsorb perrhenate ion). Prior to connection of the system to the generator, each column is wetted by passage of 20 mL of Millipore deionized water using a syringe. Once the columns are pre-wetted, they are connected as shown above using appropriate connectors and minimum-length tubing. The generator is eluted with 0.3 M ammonium acetate solution directly through the attached ion-exchange columns, with the perrhenate trapped on the QMA column. The latter resin is then washed with 20 mL of water via the syringe, and the Re-188 perrhenate desorbed with 1 mL of 0.9% sodium chloride solution.

(b) A bivalent peptide is formulated for subsequent rhenium-188 labeling, as follows: The peptide IMP 192 [Ac-Lys(DTPA)-Tyr-Lys(DTPA)-Lys(Tscg-Cys)-NH₂] shown below is to be used for the rhenium-188 labeling. For formulation, 90 mL of a solution 800 mM in sodium glucoheptonate (17.85 g, 198 mg/mL) and 100 mM sodium acetate, is prepared by adding 540 mg (514 uL) of glacial acetic acid per 90 mL portion of the glucoheptonate solution. Then, 180 mg of ascorbic acid is added per 90 mL of buffer, as an anti-oxidant. To 30 mL of this mixture is added 1 mg (6.3 x 10⁻⁷ moles) of IMP-192 peptide, followed by a 6-fold molar excess of indium chloride (1.6 mL of a 2.3 x 10⁻³ molar stock solution of indium). The indium is added to bind to the two DTPA recognition moieties, since the bispecific antibody to be used in targeting this peptide recognizes the indium-DTPA complex. To the solution is then added 90 mg of stannous chloride dihydrate, and the mixture is immediately filtered through a 0.22-micron filter, and 0.3 mL of the mixture is aliquoted into 2-mL lyophilization vials. The vials and contents, each containing 50 ug of IMP 192 peptide, are frozen using a dry ice bath, and lyophilized under vacuum.

(c) The concentrated Re-188 eluate (1 mL, 50 mCi) from the above generator elution, part 4a) is added to one of the lyophilized vials of IMP-192, part 4b) using a shielded 1-mL syringe. The vial is shaken briefly to dissolve the contents and the vial heated at 90°C for one hour. After cooling, HPLC and ITLC (instant thin-layer chromatography radioanalyses indicate a > 90% incorporation of Re-188 into the IMP 192, bound to the latter as the reduced rhenium-TscCG complex, shown below.

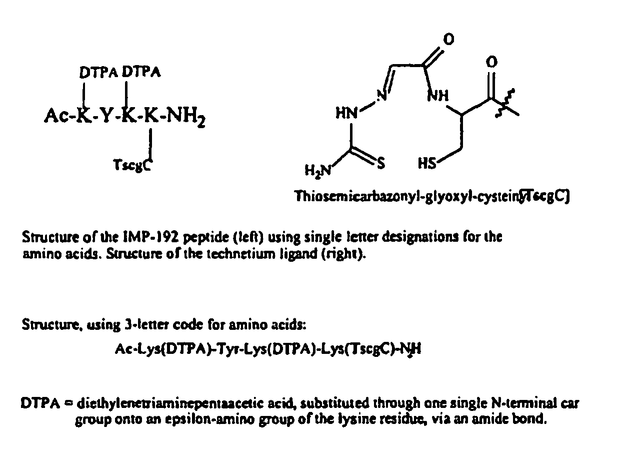

### Example 5: Preparation of a Carrier-free Rhenium-188 Radiolabeled Bivalent Hapten

The Re-188-IMP 192 from 4 c) above is diluted to 1:1 with 2 mL of degassed 200 mM phosphate buffered saline, pH 8.5, containing 5 mM EDTA. The diluted Re-188-IMP192 is added to the top of a SulfoLink^{®} coupling gel column (Pierce Chemical Co., Rockford, IL), previously equilibrated with degassed 200 mM phosphate buffered saline, pH 8.5, containing 5 mM EDTA. The Re-188-IMP 192 is allowed to run onto the gel in the column, and allowed to stand in contact with the gel for 30 minutes. After this time, the buffer containing the Re-188-IMP 192 is drained from the column, which is washed with a further 2 mL of degassed 200 mM phosphate buffered saline, pH 8.5, containing 5 mM EDTA. The Re-188-IMP 192 is then ready for injection, or is diluted further for injection or infusion.

### Example 6: Preparation of an Actinium-225 Radiolabeled Bivalent Hapten

The mono-DOTA, di-HSG bivalent hapten peptide termed IMP 241 (DOTA-Phe-Lys(HSG)-D-Tyr-Lys(HSG)-NH₂ [Figure 1] is radiolabeled with Ac-225 using ⁻ 6 nmol of peptide and ⁻ 1 mCi of dried Ac-225, such as AcCl₃. Six microliters of 0.25 M ammonium acetate, pH 5.4, followed by 2.7 uL (5.94 nmol) is added to a 2.2 mM solution of IMP-241 in 0.25 M ammonium acetate, pH 5.4. The solution is heated for one hour at 60°C using an aluminum block heater, then quenched with 10 mM DTPA (final conc.), heated for a further 10 minutes at the same temperature, and cooled. The solution is diluted with 40 uL of water, and mixed with 4.5 uL of 0.1 M aqueous triethylamine to raise the final pH to ⁻ 7.5.

### Example 7: Preparation of a Carrier-free Actinium-225 Radiolabeled Bivalent Hapten

The Ac-225-IMP 241 from Example 6, above, is purified from non-actinium-225-containing imp 241 on dowex ag 1-x2 anion exchange resin using gravity flow, using the same procedure described in example 3), above. Using this approach a 100-fold depletion in the level of non-actinium-225-containing peptide is achieved in the final product, resulting in a peptide-to-actinium-225 ratio of under 3:1. A second passage through ag 1-x2 resin reduces the peptide-to-actinium-225 ratios to very close to 1:1, if desired. The Ac-225-IMP 241 is then ready for injection, or is diluted further for injection or infusion.

### Example 8: Preparation of a High Specific Activity Radiolabeled Polymer

(a) A stirred solution of poly(L-lysine) 10 mg (about 5 x 10⁻⁸ moles; assuming an average MW of about 200,000) in 2 mL of sodium borate buffer, pH 8.5, is treated with an approximately 100-fold molar excess (⁻ 1.8 mg) of diethylenetriaminepentaacetic acid dianhydride (DTPAA; Sigma Chem.Co., St Louis, MO). After stirring for a further 15 minutes, the pH is adjusted to 4 using dropwise addition of 2 N hydrobromic acid. After a further one hour at room temperature, the mixture is dialyzed against water in a membrane having a MW cutoff of 10,000 Daltons, to remove by-products, with four changes of dialysate being made between five 3-16 h dialyses. The solution of the product is evaporated to dryness by lyophilization to recover the title compound, which is then analyzed for amino group substitution levels by the standard TNBS (trinitrobenzenesulfonic acid) assay. The product is further analyzed for DTPA chelate content by radiolabeling an accurately weighed sample with In-111/cold indium standard solution added in excess, and a determination of indium uptake versus unbound indium in the labeling mixture.

(b) the DTPA-poly-(I-lysine) as prepared in 8a), above, is radiolabeled with Y-90 using at a 1:5 ratio of Y-90 to available DTPA residues, as the latter are determined from the indium binding assay. The labeling is performed in 0.25 m ammonium acetate buffer, pH 5.4, at room temperature for fifteen minutes. The labeling mixture is then treated with an equivalent of (cold) indium chloride and allowed to stand at room temperature for a further 15 minutes. The Y-90(indium-DTPA) -poly-(I-lysine) can be purified by size exclusion chromatography to remove any excess indium ions, or can be used without further purification. The Y-90-(indium-DTPA) -poly-(I-lysine) is ready for injection, or is diluted further for injection or infusion.

### Example 9: Treatment of a Brain Cancer Patient with Premixed Bispecific Antibody-Mediated Radioimmunotherapy Using a Beta-Emitting Radionuclide

A 64-year-old male patient with recently-diagnosed glioblastome multiforme in his left hemisphere, which is limited to a focal lesion, undergoes a gross total resection, with placement of a Rickham reservoir and catheter into the surgically-created resection cavity. Placement of the catheter is confirmed by an MRI scan 24 h later. Patency of the Rickham catheter and intactness of the resection cavity created is confirmed by injecting 5 mg of In-111 conjugated to the bispecific antibody of Example 1, and obtaining gamma camera images 4, 24 and 48 h after injection. The In-111 imaging study shows restricted localization of gamma energy in the reservoir. At 4 days post surgery, the patient is treated with a 1:1 molar mixture of the bispecific antibody of Example 1, and the Y-90-IMP 241 bivalent hapten of Example 3, with Y-90 given at a dose of 12 mCi in an antibody protein dose of 10 mg by administering the premixed radioimmunotherapy agent via a needle into the reservoir. The reservoir and catheter are flushed after the antibody injection. The patient tolerates the radioimmunotherapy procedure well and is discharged 2 days later, after a post-therapy MRI scan is performed. The patient is evaluated by MRI scans every 2 months, and by the 4-month follow-up, an estimated 35% reduction in the tumor mass is observed. The patient's tumor shrinks approximately 60 percent during the nine months of follow-up without any further therapy required.

### Example 10. Treatment of a Brain Cancer Patient with Pretargeted Bispecific Antibody-Mediated Radioimmunotherapy Using a Beta-Emitting Radionuclide

A 58-year-old woman with a 3-cm temporal glioblastoma is treated surgically to remove all gross tumor. After removal of the mass, the patient is treated with an anti-tenascin x anti-indium-DTPA humanized bispecific antibody, by direct introduction of the agent into the tumor cavity, as in Example 9. After two days, to allow for localization of the bispecific antibody, which has a 20 mCi dose of 99m-Tc conjugated thereto to confirm restricted localization to the tumor lesion by gamma camera imaging at 4 and 24 h, the Re-188-IMP 192 of Example 5, above, is injected the next day, also directly into the surgical cavity. The patient tolerates the procedure well, and after have a post-therapy MRI scan two days later, she is discharged and followed every 2 months by MRI scans and various other examinations for toxicity. No adverse events are noted following therapy, and by 4 months, the 3-cm tumor appears to be reduced to a little over 1 cm in diameter.

### Example 11: Treatment of a Brain Cancer Patient with Pretargeted Bispecific Antibody-Mediated Radioimmunotherapy Using an Alpha-Emitting Radionuclide

A 48-year-old man presenting with an inoperable brain tumor in the pons, extending to the brain stem, and whose blood-brain barrier is compromised, is treated intravenously with an injection of 30 mg of humanized anti-tenascin x anti-HSG bispecific antibody. This preparation has an In-111 label to confirm targeting to the brain tumor by gamma camera imaging 24 h later. After two more days, to allow for adequate localization of the bispecific antibody to the tumor and clearance from the body, the Ac-225-IMP 241 of example 7, above, is also injected intravenously, at a dose of 1 mCi. This procedure is repeated 8 weeks later, when there is no evidence of hematological toxicity, since the patient's granulocytes (ANC) and platelets are within the normal range. At the 3-month follow-up, the patient appears to have improvement in motor functions and other neurological signs, and is generally improved. MRI of the brain at this time shows a reduction in tumor mass by about 30%, with improvement also of the patient's hydrocephalus, for which he receives prednisone at 60 mg per day. The prednisone is then gradually reduced as the hydrocephalus regressed, as confirmed by MRI. Three months later, the patient receives temozolomide therapy, which results in additional improvement and reduction of the tumor by an additional 25%. Four months later, the patient has minimal symptoms and is functionally reasonably well.

### Example 12. Treatment of a Brain Cancer Patient with Pretargeted Bispecific Antibody-Mediated Radioimmunotherapy Using an Alpha-Emitting Radionuclide

A 54-year-old man with a 2-3 cm glioblastoma multiforme in his left parietal lobe, previously treated with surgery, and whose blood-brain barrier is now compromised, is treated intravenously with an injection of 30 mg of humanized anti-tenascin x anti-HSG bispecific antibody. After two days, to allow for localization of the bispecific antibody, the Ac-225-IMP 241 of Example 7, above, at a dose of 1 mCi, is injected into the surgical cavity. The patient does not experience any adverse events from this therapy, and is discharged the next day. Four and 12 weeks later, repeat MRI scans are made to assess the extent of the brain tumor, and no progression is noted. After an additional 4 weeks, the MRI shows a decrease in the brain lesion by about 25%. After an additional 2 weeks, it is decided to enter the patient onto a chemotherapy regimen with tomozolomide, which is tolerated well. Three months later, MRI indicates that the original tumor had an approximately 60 percent regression from the baseline studies, and the patient continues to do well up to the last follow-up, at 30 weeks post radioimmunotherapy.

### Example 13. Treatment of a Brain Cancer Patient with Pretargeted Bispecific Antibody-Mediated Radioimmunotherapy Using an Alpha-Emitting Radionuclide

In an alternative treatment, the patient of Example 12 undergoes the same therapeutic regime except the bispecific antibody is injected intrathecally instead of into the surgical cavity. Results are similar to those obtained for intracavitary administration

## Claims

1. The use of (i) a multispecific antibody comprising at least one targeting arm that binds to a brain tumor antigen and at least one capture arm that binds to a radionuclide carrier and (ii) a therapeutic radionuclide carrier to which is bound a therapeutic radionuclide in the preparation of a medicament for the treatment of a brain tumor wherein the multispecific antibody, radionuclide carrier and therapeutic radionuclide are to be administered in a substantially equimolar ratio and wherein the tumor antigen is selected from the group consisting of carcinoembryonic antigen, tenascin, epidermal growth factor receptor, platelet derived growth factor receptor, fibroblast growth factor receptors, vascular endothelial growth factor receptors, gangliosides and HER2neu receptors.

2. A multispecific antibody comprising at least one targeting arm that binds to a brain tumor antigen and at least one capture arm that binds to a radionuclide carrier for use in the treatment of brain tumor, whereby the treatment comprises administering to a patient in need thereof, a pharmaceutically effective amount of
i) the multispecific antibody; and
ii) a therapeutic radionuclide carrier to which is bound a therapeutic radionuclide,
wherein the multispecific antibody, radionuclide carrier and therapeutic radionuclide are to be administered in a substantially equimolar ratio and wherein the tumor antigen is selected from the group consisting of carcinoembryonic antigen, tenascin, epidermal growth factor receptor, platelet derived growth factor receptor, fibroblast growth factor receptors, vascular endothelial growth factor receptors, gangliosides and HER2neu receptors.

3. The use of claim 1 or the multispecific antibody of claim 2, further comprising:
(a) radiolabeling a solution of the radionuclide carrier with the therapeutic radionuclide, wherein the carrier is present in excess of the therapeutic radionuclide and a therapeutic radionuclide carrier conjugate is thereby formed;
(b) substantially removing from the solution carrier that has not complexed or bound to the therapeutic radionuclide;
(c) providing the multispecific antibody; and
(d) administering sequentially or together to the patient the multispecific antibody and the radionuclide carrier conjugate;
wherein the radionuclide carrier binds to the multispecific antibody and a targeting arm or targeting arms of the multispecific antibody form an antigen-antibody complex at the target cell.

4. The use or multispecific antibody of claim 3, further comprising prior to the administering of (d):
mixing the multispecific antibody and the therapeutic radionuclide conjugate in approximately a 1:1 ratio.

5. The use or multispecific antibody of claim 3, further comprising prior to administering the therapeutic radionuclide conjugate:
administering to a patient in need thereof, intrathecaly, intracranialy, intravenously, intraarterialy, or intratumoraly, a pharmaceutically effective amount of the non-radio labeled multispecific antibody; and
allowing the multispecific antibody to bind to the tumor and to clear from circulation
administering the therapeutic radionuclide conjugate in a molar ratio of between approximately 1:10 and 1:1 with respect to the multispecfic antibody.

6. The use or multispecific antibody according to claim 3, wherein (b) is performed via an ion-exchange column.

7. The use or multispecific antibody according to claim 3, wherein (b) is performed via a size-exclusion column.

8. The use or multispecific antibody according to claim 3, wherein (b) is performed via a hydrophobic interaction column.

9. The use or multispecific antibody according to claim 3, wherein (b) is performed via a chemically activated column that is capable of reacting with a non-complexed chemical moiety on the therapeutic radionuclide carrier.

10. The use or multispecific antibody of claim 3, wherein the therapeutic radiolabled carrier comprises a metal chelating agent.

11. The use or multispecific antibody of claim 3, wherein the administration is intrathecal, intracranial, intravenous, intraarterial, or intratumoral.

12. The use of claim 1 or the multispecific antibody of claim 2, further comprising:
(a) developing the multispecific antibody so that it is specific to a conjugate of the therapeutic radionuclide and the therapeutic radionuclide carrier, wherein the multispecific antibody has an affinity for the therapeutic radionuclide carrier conjugate that is approximately 100X or more higher than its affinity for the non-radiolabeled therapeutic radionuclide carrier alone;
(b) radiolabeling a solution of the therapeutic radionuclide carrier with the therapeutic radionuclide, wherein the carrier is present in excess of the therapeutic radionuclide and a therapeutic radionuclide carrier conjugate is thereby formed;
(c) adding the multispecific antibody to the solution;
(d) optionally removing from the solution therapeutic radionuclide carrier that has not bound to the multispecific antibody; and
(e) administering the patient the coupled multispecific antibody and the therapeutic radionuclide carrier conjugate; wherein the therapeutic radionuclide carrier and the multispecific antibody are administered in approximately a 1:1 ratio.

13. The use or multispecific antibody of claim 12, wherein (d) is performed via an ion-exchange column.

14. The use or multispecific antibody of claim 12, wherein (d) is performed via a size-exclusion column.

15. The use or multispecific antibody of claim 12, wherein (d) is performed via a hydrophobic interaction column.

16. The use or multi specific antibody of claim 12, wherein (d) is performed via a chemically activated column that is capable of reacting with a non-complexed chemical moiety on the therapeutic radionuclide carrier.

17. The use or multispecific antibody of claim 12, wherein the administration is intrathecal, intracranial, intravenous, intraarterial, or intratumoral.

18. The use of claim 1 or the multispecific antibody of claim 2, further comprising:
(a) radiolabeling a solution comprising a chelate with the therapeutic radionuclide,
wherein the chelate and the radionuclide are mixed in approximately a ratio of 1:1 and a therapeutic radionuclide chelate conjugate is thereby formed;
(b) binding to the conjugate a non-radiolabled multispecific antibody;
(c) administering to the patient the coupled multispecific antibody and the therapeutic radionuclide conjugate, wherein the therapeutic radionuclide conjugate and the multispecific antibody are administered in approximately a 1:1 ratio.

19. The use or multispecific antibody of claim 18, wherein the administration is intrathecal, intracranial, intravenous, intraarterial, or intratumoral.

20. The use of claim 1 or the multispecific antibody of claim 2, further comprising:
(a) providing the therapeutic radionuclide carrier, wherein therapeutic radionuclide carrier has a thiol group;
(b) binding the thiol group of the therapeutic radionuclide carrier to a thiol-binding radionuclide, whereby a radionuclide-carrier conjugate is formed;
(c) passing the mixture of bound and unbound therapeutic radionuclide carrier over a substrate activated with a thiol-reactive chemical moiety, whereby carrier with thiol groups remaining unbound to radionuclide reacts with the substrate and is retained while therapeutic radionuclide carrier with radionuclide-bound thiol is eluted;
(d) administering to a patient in need thereof, a pharmaceutically effective amount of the multispecific antibody and the therapeutic radionuclide carrier conjugate, wherein the therapeutic radionuclide carrier conjugate binds to the multispecific antibody.

21. The use or multispecific antibody of claim 20, wherein the administration is intrathecal, intracranial, intravenous, intraarterial, or intratumoral.

22. The use or multispecific antibody of claim 21, further comprising prior to the administering of (d): mixing the multispecific antibody and the therapeutic radionuclide carrier conjugate in approximately a 1:1 ratio.

23. The use or multispecific antibody of claim 21, wherein the thiol-reactive chemical moiety is comprised of a haloacetate or a maleimide group.

24. The use or multispecific antibody of claim 21, further comprising prior to administering the therapeutic radionuclide carrier conjugate:
administering to a patient in need thereof, intrathecaly, intracranialy, intravenously, or intratumoraly, a pharmaceutically effective amount of the non-radiolabled multispecific antibody; and
allowing the multispecific antibody to bind to the tumor and to clear from circulation
administering the therapeutic radionuclide conjugate in a molar ratio of between approximately 1:10 and 1:1 with respect to the multispecific antibody.

25. The use of claim 1 or the multispecific antibody of claim 2,
wherein the multispecific antibody, the therapeutic radionuclide carrier, and the radionuclide, are administered in approximately a 1:1:1 ratio, and wherein the composition is substantially free of the therapeutic radionuclide carrier that is not bound to the radionuclide.

26. The use or multispecific antibody of claim 20, wherein the administration is intrathecal, intracranial, intravenous, intraarterial, intratumoral or into a surgically created resection cavity.

27. The use of claims 1 and 3 to 26 or the multispecific antibody of claims 2 to 26, further comprising administering a clearing agent after administering the multispecific antibody is administered.

28. The use of claims 1 and 3 to 26 or the multispecific antibody of claims 2 to 26, wherein the multispecific antibody is administered systemically and the therapeutic radionuclide carrier conjugate is administered intracranialy.

29. The use of claims 1 and 3 to 26 or the multispecific antibody of claims 2 to 26, wherein the therapeutic radionuclide carrier comprises one or more diethylenetriaminepentaacetic acid, 1, 4, 7, 10-tetraalacyclododecane-N, N', N", N"'-tetraacectic acid, and/or thiosemicarbazonyl-cysteinylglycine chelating agents.

30. The use of claims 1 and 3 to 26 or the multispecific antibody of claims 2 to 26, wherein the radionuclide is selected from the group consisting of Sc-47, Ga-67, Y-90, Ag-111, In-111, Sm-153, Tb-166, Lu-177, Bi-213, Ac-225, Cu-64, Cu-67, Pd-109, Ag-111, Re-186, Re-188, Pt-197, Bi-212, Bi-213, Pb-212, I-125, and I-131.

31. The use of claims 1 and 3 to 26 or the multispecific antibody of claims 2 to 26, wherein the therapeutic radiolabeled carrier comprises a metal chelating agent.

32. The use of claims 1 and 3 to 26 or the multispecific antibody of claims 2 to 26, wherein concomitant cancer therapies are not used.

33. The use of claim 32, wherein the concomitant cancer therapies that are not used are chemotherapy or external radiation.

34. The use of a composition comprising a pharmaceutically effective amount of
(i) a multispecific antibody comprising at least one targeting arm that binds to a brain tumor antigen and at least one capture arm that binds to a radionuclide carrier; and
(ii) a therapeutic radionuclide carrier to which is bound a therapeutic radionuclide,
for the manufacture of a medicament for treating a brain tumor, wherein the multispecific antibody, radionuclide carrier and therapeutic radionuclide are to be administered in a substantially equimolar ratio and wherein the tumor antigen is selected from the group consisting of carcinoembryonic antigen, tenascin, epidermal growth factor receptor, platelet derived growth factor receptor, fibroblast growth factor receptors, vascular endothelial growth factor receptors, gangliosides and HER2neu receptors.

## Patentansprüche

1. Verwendung (i) eines multispezifischen Antikörpers, der wenigstens einen targetierenden Arm, der an ein Gehirntumorantigen bindet, und wenigstens einen Fangarm, der an einen Radionuklidträger bindet, und (ii) einen therapeutischen Radionuklidträger umfasst, an den ein therapeutisches Radionuklid gebunden ist, bei der Herstellung eines Medikamentes für die Behandlung eines Gehirntumors, wobei der multispezifische Antikörper, der Radionuklidträger und das therapeutische Radionuklid in einem im Wesentlichen äquimolaren Verhältnis verabreicht werden sollen und wobei das Tumorantigen ausgewählt ist aus der Gruppe bestehend aus carcinoembryonalem Antigen, Tenascin, Rezeptor für epidermalen Wachstumsfaktor, Rezeptor für aus Plättchen gewonnenen Wachstumsfaktor, Rezeptoren für Fibroblastenwachstumsfaktor, Rezeptoren für vaskulären endothelialen Wachstumsfaktor, Ganglioside und HER2neu-Rezeptoren.

2. Multispezifischer Antikörper umfassend wenigstens einen targetierenden Arm, der an ein Gehirntumorantigen bindet, und wenigstens einen Fangarm, der an einen Radionuklidträger bindet, zur Verwendung bei der Behandlung von Gehirntumor, wobei die Behandlung die Verabreichung an einen Patienten, der dessen bedarf, einer pharmazeutisch wirksamen Menge
(i) des multispezifischen Antikörpers; und
(ii) eines therapeutischen Radionuklidträgers, an den ein therapeutisches Radionuklid gebunden ist, umfasst,
wobei der multispezifische Antikörper, der Radionuklidträger und das therapeutische Radionuklid in einem im Wesentlichen äquimolaren Verhältnis verabreicht werden sollen und wobei das Tumorantigen ausgewählt ist aus der Gruppe bestehend aus carcinoembryonalem Antigen, Tenascin, Rezeptor für epidermalen Wachstumsfaktor, Rezeptor für aus Plättchen gewonnenen Wachstumsfaktor, Rezeptoren für Fibroblastenwachstumsfaktor, Rezeptoren für vaskulären endothelialen Wachstumsfaktor, Ganglioside und HER2neu-Rezeptoren.

3. Verwendung nach Anspruch 1 oder multispezifischer Antikörper nach Anspruch 2, weiter umfassend:
(a) Radiomarkieren einer Lösung des Radionuklidträgers mit dem therapeutischen Radionuklid, wobei der Träger im Überschuss zum therapeutischen Radionuklid vorhanden ist, und ein therapeutisches Radionuklidträgerkonjugat **dadurch** ausgebildet wird;
(b) im Wesentlichen Entfernen des Trägers, der nicht das therapeutische Radionuklid komplexiert hat oder an dieses gebunden ist, von der Lösung;
(c) Bereitstellen des multispezifischen Antikörpers; und
(d) sequentielle oder gemeinsame Verabreichung des multispezifischen Antikörpers und des Radionuklidträgerkonjugates an den Patienten,
wobei der Radionuklidträger an den multispezifischen Antikörper bindet und ein targetierender Arm oder targetierende Arme des multispezifischen Antikörpers einen Antigen-Antikörperkomplex an der Zielzelle ausbilden.

4. Verwendung oder multispezifischer Antikörper nach Anspruch 3, weiter umfassend vor dem Verabreichen gemäß (d):
Mischen des multispezifischen Antikörpers und des therapeutischen Radionuklidkonjugates in einem Verhältnis von etwa 1:1.

5. Verwendung oder multispezifischer Antikörper nach Anspruch 3, weiter umfassend vor der Verabreichung des therapeutischen Radionuklidkonjugates:
intrathekales, intrakraniales, intravenöses, intraarterielles oder intratumorales Verabreichen einer pharmazeutisch wirksamen Menge des nicht-radioaktiv markierten multispezifischen Antikörpers an einen Patienten, der dessen bedarf; und
Erlauben, dass der multispezifische Antikörper an den Tumor bindet und aus dem Kreislauf geklärt wird;
Verabreichen des therapeutischen Radionuklidkonjugates in einem molaren Verhältnis von zwischen etwa 1:10 und 1:1 bezogen auf den multispezifischen Antikörper.

6. Verwendung oder multispezifischer Antikörper nach Anspruch 3, wobei (b) vermittels einer Ionenaustauschersäule durchgeführt wird.

7. Verwendung oder multispezifischer Antikörper nach Anspruch 3, wobei (b) vermittels einer Größenausschlusssäule durchgeführt wird.

8. Verwendung oder multispezifischer Antikörper nach Anspruch 3, wobei (b) vermittels einer hydrophoben Wechselwirkungssäule durchgeführt wird.

9. Verwendung oder multispezifischer Antikörper nach Anspruch 3, wobei (b) vermittels einer chemisch aktivierten Säule durchgeführt wird, die in der Lage ist, mit einem nichtkomplexierten chemischen Teil auf dem therapeutischen Radionuklidträger zu reagieren.

10. Verwendung oder multispezifischer Antikörper nach Anspruch 3, wobei der therapeutische radiomarkierte Träger ein Metall-komplexierendes Agens umfasst.

11. Verwendung oder multispezifischer Antikörper nach Anspruch 3, wobei die Verabreichung intrathekal, intrakranial, intravenös, intraarteriell oder intratumoral erfolgt.

12. Verwendung nach Anspruch 1 oder multispezifischer Antikörper nach Anspruch 2, weiter umfassend:
(a) Entwickeln des multispezifischen Antikörpers, so dass er für ein Konjugat aus dem therapeutischen Radionuklid und dem therapeutischen Radionuklidträger spezifisch ist, wobei der multispezifische Antikörper eine Affinität für das therapeutische Radionuklidträgerkonjugat aufweist, die etwa 100x oder mehr größer ist als seine Affinität für den nicht-radioaktiv markierten therapeutischen Radionuklidträger alleine;
(b) Radioaktives Markieren einer Lösung des therapeutischen Radionuklidträgers mit dem therapeutischen Radionuklid, wobei der Träger im Überschuss zum therapeutischen Radionuklid vorhanden ist und ein therapeutisches Radionuklidträgerkonjugat **dadurch** ausgebildet wird;
(c) Hinzugeben des multispezifischen Antikörpers zu der Lösung;
(d) optionales Entfernen des therapeutischen Radionuklidträgers, der nicht an den multispezifischen Antikörper gebunden hat, von der Lösung; und
(e) Verabreichen des gekoppelten multispezifischen Antikörpers und des therapeutischen Radionuklidträgerkonjugates an den Patienten, wobei der therapeutische Radionuklidträger und der multispezifische Antikörper in einem Verhältnis von etwa 1:1 verabreicht werden.

13. Verwendung oder multispezifischer Antikörper nach Anspruch 12, wobei (d) vermittels einer Ionenaustauschersäule durchgeführt wird.

14. Verwendung oder multispezifischer Antikörper nach Anspruch 12, wobei (d) vermittels einer Größenausschlusssäule durchgeführt wird.

15. Verwendung oder multispezifischer Antikörper nach Anspruch 12, wobei (d) vermittels einer hydrophoben Wechselwirkungssäule durchgeführt wird.

16. Verwendung oder multispezifischer Antikörper nach Anspruch 12, wobei (d) vermittels einer chemisch aktivierten Säule durchgeführt wird, die in der Lage ist mit einem nichtkomplexierten chemischen Teil auf dem therapeutischen Radionuklidträger zu reagieren.

17. Verwendung oder multispezifischer Antikörper nach Anspruch 12, wobei die Verabreichung intrathekal, intrakranial, intravenös, intraarteriell oder intratumoral ist.

18. Verwendung nach Anspruch 1 oder multispezifischer Antikörper nach Anspruch 2, weiter umfassend:
(a) radioaktives Markieren einer Lösung, die ein Chelat umfasst, mit dem therapeutischen Radionuklid, wobei das Chelat und das Radionuklid in einem Verhältnis von etwa 1:1 gemischt werden und ein therapeutisches Radionuklidchelatkonjugat **dadurch** ausgebildet wird;
(b) Binden eines nicht-radioaktiv markierten multispezifischen Antikörpers an das Konjugat;
(c) Verabreichen des gekoppelten multispezifischen Antikörpers und des therapeutischen Radionuklidkonjugates an den Patienten, wobei das therapeutische Radionuklidkonjugat und der multispezifische Antikörper in einem Verhältnis von etwa 1:1 verabreicht werden.

19. Verwendung oder multispezifischer Antikörper nach Anspruch 18, wobei die Verabreichung intrathekal, intrakranial, intravenös, intraarteriell oder intratumoral ist.

20. Verwendung nach Anspruch 1 oder multispezifischer Antikörper nach Anspruch 2, weiter umfassend:
(a) Bereitstellen des therapeutischen Radionuklidträgers, wobei der therapeutische Radionuklidträger eine Thiolgruppe aufweist;
(b) Binden der Thiolgruppe des therapeutischen Radionuklidträgers an ein Thiolbindendes Radionuklid, wobei ein Radionuklidträgerkonjugat ausgebildet wird;
(c) Geben der Mischung aus gebundenem und nicht gebundenem therapeutischen Radionuklidträger über ein Substrat, das mit einem Thiol-reaktiven chemischen Teil aktiviert ist,
wobei Träger mit Thiol-Gruppen, die an Radionuklide ungebunden bleiben, mit dem Substrat reagiert und zurückgehalten wird, wohingegen therapeutischer Radionuklidträger mit radionuklidgebundenem Thiol eluiert wird;
(d) Verabreichen einer pharmazeutisch wirksamen Menge des multispezifischen Antikörpers und des therapeutischen Radionuklidträgerkonjugates an einen Patienten, der dessen bedarf, wobei das therapeutische Radionuklidträgerkonjugat an den multispezifischen Antikörper bindet.

21. Verwendung oder multispezifischer Antikörper nach Anspruch 20, wobei die Verabreichung intrathekal, intrakranial, intravenös, intraarteriell oder intratumoral ist.

22. Verwendung oder multispezifischer Antikörper nach Anspruch 21, weiter umfassend vor der Verabreichung gemäß (d): Mischen des multispezifischen Antikörpers und des therapeutischen Radionuklidträgerkonjugates in einem Verhältnis von etwa 1:1.

23. Verwendung oder multispezifischer Antikörper nach Anspruch 21, wobei der Thiolreaktive chemische Teil aus einer Haloacetat- oder eine Maleimid-Gruppe besteht.

24. Verwendung oder multispezifischer Antikörper nach Anspruch 21, weiter umfassend vor der Verabreichung des therapeutischen Radionuklidträgerkonjugates:
intrathekales, intrakraniales, intravenöses oder intratumorales Verabreichen einer pharmazeutisch wirksamen Menge des nicht-radioaktiv markierten multispezifischen Antikörpers an einen Patienten, der dessen bedarf;
Erlauben, dass der multispezifische Antikörper an den Tumor bindet und aus dem Kreislauf geklärt wird,
Verabreichen des therapeutischen Radionuklidkonjugates in einem molaren Verhältnis von zwischen etwa 1:10 und 1:1 bezogen auf den multispezifischen Antikörper.

25. Verwendung nach Anspruch 1 oder multispezifischer Antikörper nach Anspruch 2, wobei:
der multispezifische Antikörper, der therapeutische Radionuklidträger und das Radionuklid in einem Verhältnis von etwa 1:1:1 verabreicht werden, und wobei die Zusammensetzung im Wesentlichen frei von dem therapeutischen Radionuklidträger ist, der nicht an das Radionuklid gebunden ist.

26. Verwendung oder multispezifischer Antikörper nach Anspruch 20, wobei die Verabreichung intrathekal, intrakranial, intravenös, intraarteriell, intratumoral oder in eine chirurgisch erzeugte Resektionskavität erfolgt.

27. Verwendung nach den Ansprüchen 1 und 3 bis 26 oder multispezifischer Antikörper nach den Ansprüchen 2 bis 26, weiter umfassend die Verabreichung eines Klärmittels nach Verabreichen des multispezifischen Antikörpers.

28. Verwendung nach den Ansprüchen 1 und 3 bis 26 oder multispezifischer Antikörper nach den Ansprüchen 2 bis 26, wobei der multispezifische Antikörper systemisch verabreicht wird und das therapeutische Radionuklidträgerkonjugat intrakranial verabreicht wird.

29. Verwendung nach den Ansprüchen 1 und 3 bis 26 oder multispezifischer Antikörper nach den Ansprüchen 2 bis 26, wobei der therapeutische Radionuklidträger ein oder mehrere Diethylentriaminpentaessigsäure-, 1, 4, 7, 10-Tetraazacyclododecan-N, N', N", N"'-Tetraessigsäure- und/oder Thiosemicarbazonylcysteinylglycin-komplexierende Agenzien umfasst.

30. Verwendung nach den Ansprüchen 1 und 3 bis 26 oder multispezifischer Antikörper nach den Ansprüchen 2 bis 26, wobei das Radionuklid ausgewählt ist aus der Gruppe bestehend aus Sc-47, Ga-67, Y-90, Ag111, In-111, Sm-153, Tb-166, Lu-177, Bi-213, Ac-225, Cu-64, Cu-67, Pd-109, Ag-111, Re-186, Re-188, Pt-197, Bi-212, Bi-213, Po-212, I-125 und I-131.

31. Verwendung nach den Ansprüchen 1 und 3 bis 26 oder multispezifischer Antikörper nach den Ansprüchen 2 bis 26, wobei der therapeutische radioaktiv markierte Träger ein Metall-komplexierendes Agens umfasst.

32. Verwendung nach den Ansprüchen 1 und 3 bis 26 oder multispezifischer Antikörper nach den Ansprüchen 2 bis 26, wobei keine begleitenden Krebstherapien verwendet werden.

33. Verwendung nach Anspruch 32, wobei die begleitenden Krebstherapien, die nicht verwendet werden, Chemotherapie oder Bestrahlung von außen sind.

34. Verwendung einer Zusammensetzung umfassend eine pharmazeutisch wirksamen Menge
(i) eines multispezifischen Antikörpers der wenigstens einen targetierenden Arm, der an ein Gehirntumorantigen bindet, und wenigstens einem Fangarm umfasst, der an einen Radionuklidträger bindet; und
(ii) eines therapeutischen Radionuklidträgers, an den ein therapeutisches Radionuklid gebunden ist,
für die Herstellung eines Medikamentes zur Behandlung eines Gehirntumors, wobei der multispezifische Antikörper, der Radionuklidträger und das therapeutische Radionuklid in einem im Wesentlichen äquimolaren Verhältnis verabreicht werden sollen, und wobei das Tumorantigen ausgewählt ist aus der Gruppe bestehend aus carcinoembryonalem Antigen, Tenascin, Rezeptor für epidermalen Wachstumsfaktor, Rezeptor für aus Plättchen gewonnenen Wachstumsfaktor, Rezeptoren für Fibroblastenwachstumsfaktor, Rezeptoren für vaskulären endothelialen Wachstumsfaktor, Ganglioside und HER2neu-Rezeptoren.

## Revendications

1. Utilisation de (i) un anticorps multispécifique comprenant au moins un bras de ciblage qui se lie à un antigène de tumeur cérébrale et au moins un bras de capture qui se lie à un vecteur de radionucléide et (ii) un vecteur de radionucléide thérapeutique auquel est lié un radionucléide thérapeutique dans la préparation d'un médicament pour le traitement d'une tumeur cérébrale ; l'anticorps multispécifique, le vecteur de radionucléide et le radionucléide thérapeutique devant être administrés dans un rapport pratiquement équimolaire et l'antigène tumoral étant choisi dans le groupe constitué par l'antigène carcinoembryonnaire, la ténascine, le récepteur du facteur de croissance épidermique, le récepteur du facteur de croissance dérivé des plaquettes, les récepteurs de facteurs de croissance fibroblastiques, les récepteurs du facteur de croissance endothéliale vasculaire, les gangliosides et les récepteurs HER2neu.

2. Anticorps multispécifique comprenant au moins un bras de ciblage qui se lie à un antigène de tumeur cérébrale et au moins un bras de capture qui se lie à un vecteur de radionucléide destiné à être utilisé dans le traitement d'une tumeur cérébrale, le traitement comprenant l'administration à un patient qui en a besoin d'une quantité pharmaceutiquement efficace de
(i) l'anticorps multispécifique ; et
(ii) un vecteur de radionucléide thérapeutique auquel est lié un radionucléide thérapeutique,
l'anticorps multispécifique, le vecteur de radionucléide et le radionucléide thérapeutique devant être administrés dans un rapport pratiquement équimolaire et l'antigène tumoral étant choisi dans le groupe constitué par l'antigène carcinoembryonnaire, la ténascine, le récepteur du facteur de croissance épidermique, le récepteur du facteur de croissance dérivé des plaquettes, les récepteurs de facteurs de croissance fibroblastiques, les récepteurs du facteur de croissance endothéliale vasculaire, les gangliosides et les récepteurs HER2neu.

3. Utilisation de la revendication 1 ou anticorps multispécifique de la revendication 2, comprenant en outre :
(a) le radiomarquage d'une solution du vecteur de radionucléide avec le radionucléide thérapeutique, le vecteur étant présent en excès par rapport au radionucléide et un conjugué radionucléide thérapeutique-vecteur étant de cette manière formé ;
(b) l'élimination de la solution en grande partie du vecteur qui n'a pas été complexé ou lié au radionucléide thérapeutique ;
(c) l'élaboration de l'anticorps multispécifique ; et
(d) l'administration séquentiellement ou en même temps au patient de l'anticorps multispécifique et du conjugué radionucléide-vecteur,
le vecteur de radionucléide se liant à l'anticorps multispécifique et un bras de ciblage ou des bras de ciblage de l'anticorps multispécifique formant un complexe antigène-anticorps au niveau de la cellule cible.

4. Utilisation ou anticorps multispécifique de la revendication 3, comprenant en outre avant l'administration de (d) :
le mélange de l'anticorps multispécifique et du conjugué de radionucléide thérapeutique dans à peu près un rapport de 1:1.

5. Utilisation ou anticorps multispécifique de la revendication 3, comprenant en outre avant l'administration du conjugué de radionucléide thérapeutique :
l'administration à un patient qui en a besoin, par voie intrathécale, intracrânienne, intraveineuse, intra-artérielle ou intra-tumorale, d'une quantité pharmaceutiquement efficace de l'anticorps multispécifique non radiomarqué ; et
le fait de laisser l'anticorps multispécifique se lier à la tumeur et être évacué de la circulation ;
l'administration du conjugué de radionucléide thérapeutique dans un rapport molaire compris entre à peu près 1:10 et 1:1 par rapport à l'anticorps multispécifique.

6. Utilisation ou anticorps multispécifique selon la revendication 3, (b) étant effectuée au moyen d'une colonne d'échange d'ions.

7. Utilisation ou anticorps multispécifique selon la revendication 3, (b) étant effectuée au moyen d'une colonne d'exclusion.

8. Utilisation ou anticorps multispécifique selon la revendication 3, (b) étant effectuée au moyen d'une colonne d'interaction hydrophobe.

9. Utilisation ou anticorps multispécifique selon la revendication 3, (b) étant effectuée au moyen d'une colonne chimiquement activée qui est apte à réagir avec une entité chimique non complexée sur le vecteur de radionucléide thérapeutique.

10. Utilisation ou anticorps multispécifique de la revendication 3, le vecteur radiomarqué thérapeutique comprenant un agent chélatant des métaux.

11. Utilisation ou anticorps multispécifique de la revendication 3, l'administration étant intrathécale, intracrânienne, intraveineuse, intra-artérielle ou intra-tumorale.

12. Utilisation de la revendication 1 ou anticorps multispécifique de la revendication 2, comprenant en outre :
(a) le développement de l'anticorps multispécifique de façon à ce qu'il soit spécifique pour un conjugué du radionucléide thérapeutique et du vecteur de radionucléide thérapeutique, l'anticorps multispécifique ayant une affinité pour le conjugué radionucléide thérapeutique-vecteur qui est à peu près 100x ou plus de 100x supérieure à son affinité pour le vecteur de radionucléide thérapeutique non radiomarqué seul ;
(b) le radiomarquage d'une solution du vecteur de radionucléide thérapeutique avec le radionucléide thérapeutique, le vecteur étant présent en excès par rapport au radionucléide thérapeutique et un conjugué radionucléide thérapeutique-vecteur étant de cette manière formé ;
(c) l'ajout de l'anticorps multispécifique à la solution ;
(d) facultativement l'élimination de la solution du vecteur de radionucléide thérapeutique qui ne s'est pas lié à l'anticorps multispécifique ; et
(e) l'administration au patient de l'anticorps multispécifique et du conjugué radionucléide thérapeutique-vecteur couplés, le conjugué radionucléide thérapeutique-vecteur et l'anticorps multispécifique étant administrés à peu près dans un rapport de 1:1.

13. Utilisation ou anticorps multispécifique de la revendication 12, (d) étant effectuée au moyen d'une colonne d'échange d'ions.

14. Utilisation ou anticorps multispécifique de la revendication 12, (d) étant effectuée au moyen d'une colonne d'exclusion.

15. Utilisation ou anticorps multispécifique de la revendication 12, (d) étant effectuée au moyen d'une colonne d'interaction hydrophobe.

16. Utilisation ou anticorps multispécifique de la revendication 12, (d) étant effectuée au moyen d'une colonne chimiquement activée qui est apte à réagir avec une entité chimique non complexée sur le vecteur de radionucléide thérapeutique.

17. Utilisation ou anticorps multispécifique de la revendication 12, l'administration étant intrathécale, intracrânienne, intraveineuse, intra-artérielle ou intra-tumorale.

18. Utilisation de la revendication 1 ou anticorps multispécifique de la revendication 2, comprenant en outre :
(a) le radiomarquage d'une solution comprenant un chélate avec le radionucléide thérapeutique, le chélate et le radionucléide étant mélangés dans à peu près un rapport de 1:1 et un conjugué radionucléide thérapeutiquechélate étant de cette manière formé ;
(b) la liaison au conjugué d'un anticorps multispécifique non radiomarqué ;
(c) l'administration au patient de l'anticorps multispécifique et du conjugué de radionucléide thérapeutique couplés, le conjugué de radionucléide thérapeutique et l'anticorps multispécifique étant administrés dans à peu près un rapport de 1:1.

19. Utilisation ou anticorps multispécifique de la revendication 18, l'administration étant intrathécale, intracrânienne, intraveineuse, intra-artérielle ou intra-tumorale.

20. Utilisation de la revendication 1 ou anticorps multispécifique de la revendication 2, comprenant en outre :
(a) l'élaboration du vecteur de radionucléide thérapeutique, le vecteur de radionucléide thérapeutique ayant un groupe thiol ;
(b) la liaison du groupe thiol du vecteur de radionucléide thérapeutique à un radionucléide se liant aux thiols, ce par quoi un conjugué radionucléide-vecteur est formé ;
(c) le passage du mélange de radionucléide thérapeutique et de vecteur liés et non liés sur un substrat activé avec une entité chimique réactive vis-à-vis des thiols, ce par quoi un vecteur avec des groupes thiols restant non liés au radionucléide réagit avec le substrat et est retenu alors que le vecteur de radionucléide thérapeutique avec un thiol lié au radionucléide est élué ;
(d) l'administration à un patient qui en a besoin d'une quantité pharmaceutiquement efficace de l'anticorps multispécifique et du conjugué radionucléide thérapeutique-vecteur, le conjugué radionucléide thérapeutique-vecteur se liant à l'anticorps multispécifique.

21. Utilisation ou anticorps multispécifique de la revendication 20, l'administration étant intrathécale, intracrânienne, intraveineuse, intra-artérielle ou intra-tumorale.

22. Utilisation ou anticorps multispécifique de la revendication 21, comprenant en outre avant l'administration de (d) : le mélange de l'anticorps multispécifique et du conjugué radionucléide thérapeutique-vecteur dans à peu près un rapport de 1: 1.

23. Utilisation ou anticorps multispécifique de la revendication 21, l'entité chimique réactive vis-à-vis des thiols étant constituée d'un haloacétate ou d'un groupe maléimide.

24. Utilisation ou anticorps multispécifique de la revendication 21, comprenant en outre avant l'administration du conjugué radionucléide thérapeutique-vecteur :
l'administration à un patient qui en a besoin, par voie intrathécale, intracrânienne, intraveineuse ou intra-tumorale, d'une quantité pharmaceutiquement efficace de l'anticorps multispécifique non radiomarqué ; et
le fait de laisser l'anticorps multispécifique se lier à la tumeur et être évacué de la circulation ;
l'administration du conjugué de radionucléide thérapeutique dans un rapport molaire compris entre à peu près 1:10 et 1:1 par rapport à l'anticorps multispécifique.

25. Utilisation de la revendication 1 ou anticorps multispécifique de la revendication 2, l'anticorps multispécifique, le vecteur de radionucléide thérapeutique et le radionucléide étant administrés dans à peu près un rapport de 1:1:1 et la composition étant pratiquement exempte du vecteur de radionucléide thérapeutique qui n'est pas lié au radionucléide.

26. Utilisation ou anticorps multispécifique de la revendication 20, l'administration étant intrathécale, intracrânienne, intraveineuse, intra-artérielle, intra-tumorale ou dans une cavité de résection créée chirurgicalement.

27. Utilisation des revendications 1 et 3 à 26 ou anticorps multispécifique des revendications 2 à 26, comprenant en outre l'administration d'un agent de clairance après l'administration de l'anticorps multispécifique.

28. Utilisation des revendications 1 et 3 à 26 ou anticorps multispécifique des revendications 2 à 26, l'anticorps multispécifique étant administré de façon systémique et le conjugué radionucléide thérapeutique-vecteur étant administré par voie intracrânienne.

29. Utilisation des revendications 1 et 3 à 26 ou anticorps multispécifique des revendications 2 à 26, le vecteur de radionucléide thérapeutique comprenant un ou plusieurs agents chélatants de type acide diéthylènetriaminepentaacétique, acide 1,4,7,10-tétraazacyclododécane-N,N',N",N"'-tétraacétique et/ou thiosemicarbazonylcystéinylglycine.

30. Utilisation des revendications 1 et 3 à 26 ou anticorps multispécifique des revendications 2 à 26, le radionucléide étant choisi dans le groupe constitué par Sc-47, Ga-67, Y-90, Ag-111, In-111, Sm-153, Tb-166, Lu-177, Bi-213, Ac-225, Cu-64, Cu-67, Pd-109, Ag-111, Re-186, Re-188, Pt-197, Bi-212, Bi-213, Pb-212, 1-125 et 1-131.

31. Utilisation des revendications 1 et 3 à 26 ou anticorps multispécifique des revendications 2 à 26, le vecteur radiomarqué thérapeutique comprenant un agent chélatant des métaux.

32. Utilisation des revendications 1 et 3 à 26 ou anticorps multispécifique des revendications 2 à 26, des thérapies anticancéreuses concomitantes n'étant pas utilisées.

33. Utilisation de la revendication 32, les thérapies anticancéreuses concomitantes qui ne sont pas utilisées étant une chimiothérapie ou un rayonnement externe.

34. Utilisation d'une composition comprenant une quantité pharmaceutiquement efficace de
(i) un anticorps multispécifique comprenant au moins un bras de ciblage qui se lie à un antigène de tumeur cérébrale et au moins un bras de capture qui se lie à un vecteur de radionucléide ; et
(ii) un vecteur de radionucléide thérapeutique auquel est lié un radionucléide thérapeutique,
pour la fabrication d'un médicament pour traiter une tumeur cérébrale, l'anticorps multispécifique, le vecteur de radionucléide et le radionucléide thérapeutique devant être administrés dans un rapport pratiquement équimolaire et l'antigène tumoral étant choisi dans le groupe constitué par l'antigène carcinoembryonnaire, la ténascine, le récepteur du facteur de croissance épidermique, le récepteur du facteur de croissance dérivé des plaquettes, les récepteurs de facteurs de croissance fibroblastiques, les récepteurs du facteur de croissance endothéliale vasculaire, les gangliosides et les récepteurs HER2neu.
